# EUROPEAN PATENT SPECIFICATION

(11) **EP 0 626 864 B1**
(45) Date of publication and mention of the grant of the patent: **02.07.2003**
(21) Application number: 93903755.2
(22) Date of filing: 16.02.1993
(51) Int. Cl.: A61K 47/36

(54) **TOPICAL COMPOSITION CONTAINING HYALURONIC ACID AND NSAIDS**
TOPISCH ANZUWENDENDES ARZNEIMITTEL ENTHALTEND HYALURONSÄURE UND NSAIDS
COMPOSITION A USAGE LOCAL CONTENANT DE L'ACIDE HYALURONIQUE ET DES ANTI-INFLAMMATOIRES NON STEROIDIENS

(30) Priority: 20.02.1992 CA 2061566
(43) Date of publication of application: 07.12.1994
(73) Proprietor: JAGOTEC AG, 4132 Muttenz (CH)
(72) Inventor: FALK, Rudolf, Edgar, Toronto, Ontario M4N 3C7 (CA); ASCULAI, Samuel Simon, Toronto, Ontario M6M 2B6 (CA)
(74) Representative: Bassil, Nicholas Charles
(86) International application number: CA9300062
(87) International publication number: WO93016733

(56) References cited:
- EP-A- 0 197 718
- EP-A- 0 368 253
- WO-A-91/04058
- US-A- 4 937 254

## Description

### FIELD OF INVENTION

This invention relates to the use of hyaluronic acid and/or salts thereof in the manufacture of pharmaceutical compositions for the treatment of disease and conditions of the skin and exposed tissue, for example, basal cell carcinoma, squamous cell tumours, metastatic cancer of the breast to the skin, primary and metastatic melanoma in the skin, malignancies and tumours in the skin, genital warts (condyloma acuminata), cervical cancer, HPV (Human Papilloma Virus) including HPV (Human Papilloma Virus) on the cervix, psoriasis (both plaque-type psoriasis and nail bed psoriasis), corns on the feet, actinic keratoses lesions, "liver" spots, fungal lesions, and other such types of lesions, and hair loss on the head of a pregnant women.

### BACKGROUND OF THE INVENTION

Basal cell carcinoma is presently treated by surgery. Each lesion, together with all surrounding and underlying tissue (dermis, epidermis and subdermis), is cut out. In some instances, surgery, while necessary for the patient's welfare, puts the patient at risk in some other respect (for example, the removal of a lesion on a patient's temple (resection) may jeopardize the patient's health). Squamous cell tumours are also treated the same way as are other forms of cancer in the skin and exposed tissue. Furthermore, other conditions and diseases of the skin and exposed tissue are treated in the same way or in ways that cause discomfort to the patient, for example, melanoma, genital warts, cervical cancer, HPV (Human Papilloma Virus).

Actinic keratoses lesion is dealt with similarly. Liquid nitrogen is also used to remove the lesion.

These diseases and conditions are usually found in the epidermis (at least for the most part, extending into the dermis and upwards through the Stratum Corneum).

Hyaluronic acid is a naturally occurring glycosaminoglycan. Its molecular weight may vary from 50,000 daltons upwards, and it forms highly viscous solutions. As regards the actual molecular weight of hyaluronic acid in natural biological contexts, this is still a matter of much uncertainty; when the molecular weight of hyaluronic acid is to be determined, different values are obtained depending on the assay method employed, and on the source, the isolation method etc. The acid occurs in animal tissue, e.g. spinal fluid, ocular fluid, synovial fluid, cockscombs, skin, and also in some streptococci. Various grades of hyaluronic acid have been obtained. A preparation with an allegedly high degree of purity and alleged to be entirely free from side effects, is a non-inflammatory form described in U.S. Patent No.4,141,973; this preparation is said to have a molecular weight exceeding 750,000 dalton, preferably exceeding 1,200,000 dalton and is suggested for therapeutic use in various articular conditions. Applicants believe that hyaluronic acid claimed in this patent is sold under the trade mark Healon.

United States Patent 4,801,619 relates to hyaluronic acid,having a molecular weight of about 3 X 10⁶ dalton or more, administered intra-articularly which is prone to decrease the proteoglycan content of synovial fluid to almost normal levels. According to this patent, this indicates a positive effect on the proteoglycan metabolism of a joint. According to the patent, this is applicable both to inflammatory conditions and to degeneration caused by treatment with symptomatics, such as corticosteroid preparations. It is thus clear that a sufficiently high molecular weight of the hyaluronic acid is alleged to counteract side effects that might be caused by corticosteroids or other symptomatics producing similar effects. When corticosteroids are applied, the amount of hyaluronic acid in the synovial cavity will, according to the patent, increase substantially and, according to the inventors, their hyaluronic acid preparations have a very positive effect on such clinical symptoms as pain, swelling, and lameness.

The patent states that the objectives of the invention are attained by intra-articular administration (injection) of an effective amount of hyaluronic acid with a mean molecular weight exceeding 3 X 10⁶ dalton, preferably exceeding 4 X 10⁶ dalton; usually the molecular weight will not exceed 7 X 10⁶ dalton. The dosage of hyaluronic acid administered is stated to be preferably within the range of 5mg-80mg. The amount of solution given at each administration is generally less than 60 ml, e.g. less than 20 ml. of an aqueous solution of the acid or its salt. It is convenient to administer the acid dissolved in water (<2% w/w, buffered to physiological pH), for instance in the form of a water-soluble sodium salt. The exact amount will depend on the particular joint to be treated.

The Merck Index Specifies that Hyaluronic Acid has a Molecular Weight within the range pf 50,000 to 8 X 10⁶ depen ding on source, methods of preparation, and methods of determination. The Merck Publication teaches hyaluronic acid as a surgical aid (ophthalmological).

United States Patent 4,808,576 purports to teach that hyaluronic acid, an agent well known for reducing the sequelae of trauma in mammalian joint tissue when injected directly into the traumatized joint tissue, will be carried to such traumatized tissue by the mammal's natural processes if applied at a site remote from the traumatized tissue. Thus, hyaluronic acid in any therapeutically acceptable form can, according to the Patent, be administered by the typical remote routes including intravenous, intramuscular, subcutaneous, and topical.

This, the patent alleges, makes the utilization of hyaluronic acid much more convenient and attractive. For instance, the treatment of arthritis in horse or human joints with hyaluronic acid, according to the patent, no longer requires more difficult intra-articular injections.

United States Patent 4,725,585 relates to a method of enhancing or regulating the host defence of a mammal by administering to a mammal a therapeutically effective amount of hyaluronic acid.

At column 1, lines 43 - 46, the patent provides that the invention was based on the unexpected discovery that administration of hyaluronic acid to mammals results in a considerable increase in the defence.

The hyaluronic acid employed in the patent was Healon (t.m) provided by Pharmacia AB, Uppsala, Sweden (Pharmacia AB is also entitled to the benefit of United States Patent 4,141,973). The patent provides at column 4, line 19 that because a patient's infections had been hard to treat, instead of just hyaluronic acid being administered to the patient to increase the patient's defence, the patient was given hyaluronic acid and an antibiotic. While one reading the patent may conclude that the antibiotic was given in combination with hyaluronic acid, in fact because the hyaluronic acid was administered subcutaneously and because the patient was a heart patient, one skilled in the art would understand that any antibiotic administered, while possibly administered simultaneously with the administration of the hyaluronic acid, was definitely administered separately intravenously (probably) or intramuscularly (less probably). Thus, the hyaluronic acid administered, according to the teachings of this patent, was administered in order to prevent possible development of infections (increase the host's defence) and not for any other reason.

United States Patent 4,636,524 discloses cross-linked gels of hyaluronic acid, alone and mixed with other hydrophilic polymers and containing various substances or covalently bonded low molecular weight substances and processes for preparing them. These products are alleged to be useful in numerous applications including cosmetic formulations and as drug delivery systems.

The patent further states that as hyaluronic acid is known to be a biologically tolerable polymer in the sense that it does not cause any immune or other kind of response when introduced into a human body, the cross-linked hyaluronic acid gels can be used for various medical applications. The cross-linked gels modified with other polymers or low molecular weight substances, it is alleged, can be used as drug delivery devices. For example, the inventors are alleged to have found that heparin introduced in a cross-linked hyaluronic acid gel retained its antithrombogenic activity.

The inventors also allege that they have also found that cross-linked gels of hyaluronic acid can slow down the release of a low molecular weight substance dispersed therein but not covalently attached to the gel macromolecular matrix.

Unites States Patent 4,736,024 purports to teach new medicaments for topical use containing:
(i) an active pharmacological substance or a mixture of pharmacological substances, either active or suitable for topical administration and
(ii) a topical vehicle which comprises hyaluronic acid or a molecular fraction of hyaluronic acid or a salt of the same with an alkaline metal, an alkaline earth metal, magnesium, aluminium, ammonium, or a pharmacological substance optionally together with additional conventional excipients for pharmaceutical preparations for topical use.

Applicants are also aware of published Japanese Patent Document 61000017, dated 86/01/06, whose English abstract of disclosure states that the Japanese Patent Document relates to the use of hyaluronic acid or cross-linked hyaluronic acid or their salts as the active ingredient for inhibiting carcinoma metastasis.

According to the purported abstract of the patent, more that 1.0% of hyaluronic acid is dissolved in alkaline aq. soln. and pref. more than 50% of H₂O sol. org. solvent. eq. alcohol, acetone, dioxane, against total soln. is added. Preferably the pH is 12-14. Then a multifunctional epoxy cpd. is added and reacted at 10-60 deg. C, pref. at 20-40- deg. C for 24 hrs. Cross-linking ratio of crosslinked hyaluronic acid or its salt is regulated by changing mol ratio of hyaluronic acid or its salt and multifunctional epoxy cpd.. Pref. hyaluronic acid used has intrinsic viscosity 0.2-30,m.w. 4000-2000000. The hyaluronic acid is allegedly used in several dosage forms. The clinical dose for an adult is alleged to be normally, as hyaluronic acid or cross-linked hyaluronic acid, 25mg-5 g/day (p.o.) and 10 mg-2.5 g/l dose (inj). The abstract alleges that the advantage is that the hyaluronic acid has no side effects as may other anti-cancer drugs and has an analgesic and a tissue restoration effect.

European Patent Application 0295092 purports to teach a vehicle together with fragments of hyaluronic acid for delivering of the fragments of hyaluronic acid into the skin to reach the dermal layer of the skin to increase the development of blood vessels for stimulating hair growth or regrowth. The preferred fragments of hyaluronic acid are polysaccharides containing from 7 to 25 monosaccharide units. The patent provides that it is apparent that the larger the fragments of hyaluronic acid, the greater the difficulty there is in delivering the fragments to the dermal layer of the skin, unless there is also present in the composition a means for enhancing the activity of said fragments.

The combination may thus include a means for enhancing the activity of the fragments of hyaluronic acid, especially to improve their penetration through the skin following topical application. Some activity enhancers, it is alleged, also function as vehicles for the fragments of the hyaluronic acid.

Some activity enhancers are also alleged to possess the ability to stimulate or increase hair growth. Minoxidil is asserted among others to be such an activity enhancer. Thus both the fragments of hyaluronic acid and minoxidil are alleged to stimulate hair growth both delivered by a vehicle.

European Patent Application 0179442 asserts that where free radicals are formed in considerable quantities, hyaluronic acid is broken down or degraded before the hyaluronic acid has given the desired effect.

Canadian Letters Patent 1,240,929 teaches the combination of chondroitin sulfate compound and a hyaluronate to protect both human and animal cell layers and tissue subject to exposure to trauma.

European Patent Application 0208623 purports to teach hyaluronic acid as "une augmentation de l'activité de certaines proteases". It also purports to teach the use of hyaluronic acid for treating connective tissue diseases, including malignant tumours and cardiovascular disorders.

European Patent Application 270317 purports to teach the combination of an antiviral agent lacking inhibitory action and a compound [for example, hyaluronic acid] possessing cell fusion inhibitory activity and/or virus-adsorption inhibitory activity for treating disease carried by a virus.

United States Patent 4,840,941 purports to teach the use of an effective amount of hyaluronic acid as the active agent for the treatment of retroviruses in association with a pharmaceutically acceptable carrier, diluent, or excipient.

United States Patent 4,851,521 and European Patent Application 0265116 both describe hyaluronic acid fractions, the making thereof and cross-linked esters of hyaluronic. United States Patent 4,851,521 describes esters of hyaluronic acid incorporated into pharmaceutical preparations as the active ingredient and as vehicles for ophthamological medicines for topical use (See column 11, lines 35 to 42; and column 12, lines 62 to column 13, line 3) and in suppositories for a systemic effect due to the effect of transcutaneous absorption, such as in suppositories.

The patent provides at column 13, lines 5 to 31:
"The vehicling action of the hyaluronic esters also applies to associated medicaments of the type mentioned above in which the active substance acts not only topically or by nasal or rectal absorption, for example by nasal sprays or preparations for inhalation for the oral cavity or the pharynx, but also by oral or parenteral route, for example by intramuscular, subcutaneous or intravenous route, as it favors absorption of the drug into the application site. The new medicaments can therefore be applied, apart from in the fields already mentioned, in practically all sectors of medicine, such as internal medicine, for example in pathologies of the cardiovascular system, in infections of the respiratory system, the digestive system, the renal system, in diseases of an endocrinological nature, in oncology, in psychiatry etc., and may also be classified therefore from the point of view of their specific action, being perhaps anesthetics, analgesics, anti-inflammatories, wound healers, antimicrobics, adrenergic agonists and antagonists, cytostatics, antirheumatics, antihypertensives, diuretics, sexual hormones, immunostimulants and immunosuppressants, for example, one of the drugs having the activity already described for the therapeutically active alcohols to be used as esterifying component according to the present invention, or for the therapeutically active bases used for the salification of the free carboxylic groups."

There have been extensive studies to determine the defect in immune function that allows a tumour cell to develop. It was postulated initially by Jerne, and subsequently by Burnett, that the immune system's major role was that of immunological surveillance to destroy abnormal cells. The concept of surveillance, while somewhat simplistic, remains an accepted concept for the elaborate mechanism of immune recognition and function that is present in the higher species - mammals.

It has then been postulated that tumours develop because of local or generalized immune suppression. However, as pointed out by Moller, if general immune suppression occurs, it is only certain types of neoplastic disorders that develop, mainly those of the lympho-reticular system. This observation is generally correct and represents a major challenge to the immune surveillance theory unless a specific reason can be shown as to why the individual cancer cell can develop plus individually evade the immune system.

It was demonstrated experimentally in 1974 that defects of macrophage function may exist in neoplastic disease.

The initial experiments found suppressor cells to be part of the immune system; these were either of the T-cell type of the macrophage cell system. There was presence demonstrated in neoplasia, chronic bacterial infection, recovery from massive injury and chronic fungal infection.

There has been repeated demonstration in experimental animals that the macrophage cell function is altered in neoplastic disease. The macrophages in the animal's systems appeared "blocked" in their function. Generally when removed from the *in vivo* situation, washed in saline and cultured, they perform normally. This block has been shown to be related to the excessive production of prostaglandin by neoplastic tissue or by the macrophage itself. Similarly, the N.K. cells (which are said to be primitive or immature macrophages and which may be involved in cancer defence) are also blocked.

In the basic research efforts in the latter '70s and the early '80's, there existed considerable confusion as tc what role immunotherapy should take in cancer. Activation or "hyping" of macrophages was thought to be important. However, in an examination by Romans and Falk of peritoneal macrophages obtained from patients with neoplastic disease, there was definite evidence that these macrophages were already activated yet were co-existing with cancer cells and not causing their destruction.

It has recently been shown by several independent investigators that the malfunction of macrophages or the putitive block is due to excessive prostaglandin and that this can be altered in tissue culture by corticosteroids, ASA, and the non-steroidal anti-inflammatory drugs, i.e. indomethacir and naproxen (Naprosyn™). Again, it was repeatedl demonstrated that in animal tumours these substances could alter the response to neoplastic cells and that various combinations of these substances employed with immune enhancing agents could produce very credible success in eliminating experimental tumours. Lala and co-workers combined Indomethacin therapy with Interleukin 2 and showed that this could effect a cure with experiment neoplasm.

There were continued problems with the use of any of these agents in the actual human *in vivo* experience. All of the non-steroidal anti-inflammatory agents (NSAID) produced major toxicity in terms of gastro-intestinal, neurological, and other areas. Thus, the basis of the present approach is that, under general circumstances, with the use of these agents in human disease in sufficient amounts, the drug will penetrate to any pathological tissue to alter therapeutically local prostaglandin production. While intravenous preparations of Indomethacin (and now of other agents) exist, using these drugs alone produces prohibitive side effects in human subjects. Therefore, only insufficient amounts can be brought into the body to effect more than occasional responses in neoplasm.

However, the majority of the evidence is present to indicate and therefore, it can be postulated that the basis for neoplastic development and how the initial cell "sneaks by" the immune surveillance mechanism relates to its production of prostaglandin. One need postulate only one mutation to alter the amount of prostaglandin synthesis produced by cells when they become "malignant" to establish a mechanism of blocking out the initial cell in any immune reaction, i.e. the macrophage. It therefore became essential to develop a combination of NSAIDs for clinical use to produce a major improvement in response in neoplastic disease and other conditions where excessive prostaglandin synthesis represents the basis of the pathogenesis of this disease state, i.e. arthritis and various others of the so-called connective tissue inflammatory disorders and/or auto-aggressive diseases.

See also:
1. Modulation of Immunity in Cancer Patients by Prostaglandin Antagonists, Immunity to Cancer II, Alan R. Liss, Inc.; and
2. Goodwin, J.S., (1981) Prostaglandin E and Cancer Growth Potential for Immunotherapy with Prostaglandin Synthesis Inhibitors, Augmentive Agents in Cancer Therapy, Raven Press, New York.

United States Patent 4,711,780 teaches a pharmaceutical composition comprising Vitamin C, a zinc salt, and a sulfur amino acid for treating surface epithelium for epithelium regeneration. Hyaluronic acid may be added for applications in the reproductive tract to block the passage of toxins into the blood system.

U.S. Patent 4,937,254 (Ethicon) teaches combinations of hyaluronic acid and salts thereof with NSAIDS for the prevention of adhesions after surgery.

EP-A-0197718 discloses compositions for topical administration comprising a therapeutically active drug and hyaluronic acid or derivatives thereof, but does not describe or indicate how to formulate such compositions for percutaneous transport of the drug or for accumulation and retention of the drug at a desired target site.

WO-A-91/04058 describes compositions comprising a therapeutic agent and a form of hyaluronic acid in an amount sufficient to facilitate the agent's penetration through skin, but does not describe accumulating or retaining the agent at a desired target site.

Because of the side effects of the use of non-steroidal anti-inflammatory drugs (major toxicity in terms of gastro-intestinal, neurological, and other areas, use thereof should also be restricted (if possible) to the area of use without delivery to other areas which are not in need of treatment. Thus, if useful amounts of the non-steroidal anti-inflammatory drugs or for that matter any drugs could be delivered to a site in need thereof without carriage of substantial amounts away from the site to be treated, thereby accumulating an amount of the drug at the site to be treated for a prolonged period of time, then the use of the drug for example a non-steroidal anti-inflammatory drug at a site may have many other useful applications.

### SUMMARY OF THE INVENTION

According to the invention, there are provided the uses set out in the Claims, in which hyaluronic acid and/or salts thereof and/or homologues, analogues, derivatives, complexes, esters, fragments and subunits of hyaluronic acid are applied in a dosage amount exceeding 5g/cm² of skin or exposed tissue.

The uses of the invention are now further defined with reference to compositions and embodiments resulting from the uses.

Applicants have thus now developed uses of compositions according to the claims (combinations and formulations) which are topically applied to the skin and/or exposed tissue of a human and which are quickly transported in dosage amounts percutaneously (intracutaneously) at a site in need of treatment, (site of pathology and/or trauma) best targeting the epidermis and subsequently remaining (accumulating) at the site for a prolonged period of time. The compositions subsequently clear through the lymphatics thereby bringing dosage amounts of the compositions to the lymphatics for the treatment of disease and conditions in the lymphatics.

These compositions, (combinations and formulations) employ, combine, or incorporate (as the case may be) a plurality of effective non-toxic dosage amounts, each dosage amount comprising an effective non-toxic amount of a drug for example a drug which inhibits prostaglandin synthesis for example an NSAID and an effective non-toxic dosage amount of a form of hyaluronic acid (preferably hyaluronic acid or salt thereof) for the transport of the drug to the site of the pathology and/or trauma. Suitable dosage amounts of the composition may be removed from a container (for example a tube or jar) and administered (for example, applied).

These pharmaceutical compositions (combinations and formulations) comprise a plurality of effective non-toxic dosage amounts for administration to the skin and/or exposed tissue of a human in need of treatment, each such dosage amount comprising a therapeutically effective non-toxic (to the patient) dosage amount of a drug to treat a disease and/or condition for example a drug which inhibits prostaglandin synthesis, preferably being a non-steroidal anti-inflammatory drug (NSAID), for example, diclofenac, indomethacin, naproxen, and (+/-) tromethamine salt of ketorolac (sold under the trademark Toradol™) and an effective non-toxic dosage amount in excess of 5 mg per cm² (square centimeter) of skin or exposed tissue to which the dosage amount of the composition is to be applied of hyaluronic acid and/or salts thereof (for example the sodium salt) and/or homologues, analogues, derivatives, complexes, esters, fragments, and/or subunits of hyaluronic acid (preferably hyaluronic acid and/or salts thereof) to transport (to facilitate or cause the transport of) the drug to the site of the pathology and/or trauma of the disease or condition. These compositions are applied topically to treat diseases and conditions of the skin and/or exposed tissue at the site of the trauma and/or pathology, (for example, basal cell carcinoma, the precancerous, often recurrent, actinic keratoses lesions, fungal lesions, "liver" spots and like lesions (found for the most part in the epidermis), squamous cell tumours, metastatic cancer of the breast to the skin, primary and metastatic melanoma in the skin, malignancies and/or tumours in the skin, genital warts (condyloma acuminata), cervical cancer, and HPV (Human Papilloma Virus) including HPV of the cervix, psoriasis (both plaque-type psoriasis and nail bed psoriasis), corns on the feet and hair loss on the head of pregnant women. The results of the treatment with suitable dosage amounts taken from these compositions (combinations and formulations) have been in some instances quite dramatic - difficult situations have been successfully treated and resolved.

Furthermore, application of the dosage amounts of the compositions, combinations and formulations are, systemic independent (there is a lack of a blood level of the drug for example NSAID), are quick to penetrate into the skin to the site of the trauma and/or pathology because the effective dosage amount of the form of hyaluronic acid transports (facilitates or causes the transport of) the drug (for example NSAID) particularly to the epidermis where the composition, combination or formulation accumulates and remains for prolonged periods. The compositions subsequently clear through the lymphatics and are available for the treatment of disease and conditions of the lymphatics.

Effective dosage amounts of the form of hyaluronic acid to facilitate or cause the transport of the drug into the skin and/or exposed tissue by the form of hyaluronic acid exceeds 5 mg - 10 mg in the dosage amount administered (applied and rubbed in) for each 1 cm² of skin and/or exposed tissue area of the disease or condition (for example basal cell carcinoma) to which the dosage amount is applied. The dosage amount applicable will depend upon the surface area of the skin and/or exposed tissue in which the condition or disease exists. Preferred forms of the hyaluronic acid (for example hyaluronic acid and the sodium salt thereof) have molecular weights less than about 750,000 daltons (for example about 150,000 to about 225,000 daltons) to transport the medicine in the skin and/or exposed tissue. While higher molecular weights of the hyaluronic acid and forms thereof may be used to penetrate the skin and/or exposed tissue and transport the medicines or drugs, where the molecular weight of the hyaluronic acid chosen for use is very large, it is preferred that the form of hyaluronic acid is autoclaved, to break down the hyaluronic acid to fragments of lesser molecular weight or if feasible diluted to permit administration and ensure no coagulation on or in the skin. Where the molecular weight of the form of hyaluronic acid being employed is large, the concentration of the form of the hyaluronic acid in the composition may for example be reduced (for example to less than about 3%) dependent on molecular weight.

The blockage of prostaglandin synthesis by the transported drug (for example NSAIDS) then unblocks the macrophages and permits the macrophages of the patient proximate the lesion (for example, the basal cell carcinoma) to destroy the lesion or condition. Treatment by dosage amounts of the composition (formulation and/or combination) eliminates the condition without recurrence, even where the lesion has recurred a number of times after unsuccessful treatments according to the prior art.

Other non-steroidal anti-inflammatory drugs (NSAIDS) may be used such as other propionic acid derivatives, Ibuprofen, acetylsalicylic acid, piroxicam and flunixin.

When dosage amounts of such compositions, combinations and formulations are applied to the site of the disease or conditions for example the basal cell carcinoma of the patient suffering from the basal cell carcinoma, over a period of time (for example, for a period of 2-4 weeks 3 times daily) the basal cell carcinoma is completely resolved and disappears.

Therefore, according to the invention there is provided the use of:
(1) a medicinal and/or therapeutic agent in a therapeutically effective amount to treat a disease or condition of the skin and/or exposed tissue; and
(2) hyaluronic acid and/or salts thereof and/or homologues, analogues, derivatives, complexes, esters, fragments, and subunits of hyaluronic acid,
characterized in that said composition is topically applied in a dosage amount in which component (2) exceeds 5 mg/cm² of the skin and/or exposed tissue to which the dosage amount is to be applied and component (2) is immediately available to transport (facilitate or cause the transport of) component (1) to the site of trauma and/or pathology to be treated, percutaneously into the skin (or exposed tissue). Preferably the form of hyaluronic acid in the composition comprises hyaluronic acid and/or salts thereof. An effective amount of the form of hyaluronic acid exceeds 5-10 mg per square centimeter (cm²) of skin and/or exposed tissue to which it is to be applied.

The pharmaceutical composition will normally include pharmaceutically compatible excipients to provide a form for ease of administration to the skin and/or exposed tissue for transport into the epidermis. For example a suitable dosage amount of a gel may be squeezed from a tube as a ribbon of gel "X" cm long (which dosage amount (in the form of the ribbon "X" cm long) contains the effective non-toxic dosage amounts of the drug and form of hyaluronic acid. Or a dosage amount of cream packaged in a jar may be scooped from the jar by a measuring device or by "two fingers" in a suitable amount (for example in a spoon containing a premeasured volume or an amount about half the "length of the fingers"). Each of the dosage amounts selected comprises the effective amounts of drug (for example NSAID) and effective amount of the form of hyaluronic acid (for example hyaluronic acid and/or salts thereof). In this way the patient may "squeeze" or "scoop" or "what have you" the appropriate dosage amount and apply (rub in) the dosage amount onto the skin and/or exposed tissue for transport into the epidermis.

A disease and/or condition of the skin or exposed tissue, includes for example basal cell carcinoma, the precancerous, often recurrent, actinic keratoses lesions, fungal lesions, "liver" spots and like lesions (found for the most part in the epidermis), squamous cell tumours, metastatic cancer of the breast to the skin, primary and metastatic melanoma in the skin, malignancies and/or tumours in the skin, genital warts (condyloma acuminata), cervical cancer, HPV (Human Papilloma Virus) including HPV of the cervix, psoriasis (both plaque-type psoriasis and nail bed psoriasis), corns on the feet and hair loss on the head of pregnant women, in a human. For example, the composition may be in the form of a gel or cream (to give the composition definition and form so that specific dosage amounts are easily selected or taken for administration (for example squeezed from a tube or scooped from a jar and rubbed into the skin or exposed tissue), a therapeutically effective (to treat and to assist to resolve the disease or condition for example basal cell carcinoma or other lesion), non-toxic (to the patient) dosage amount of a drug for example which inhibits prostaglandin synthesis, for example a non-steroidal anti-inflammatory drug (NSAID), for example, diclofenac, indomethacin, naproxen, and (+/-) tromethamine salt of ketorolac (sold under the trademark Toradol™) and an effective non-toxic dosage amount of hyaluronic acid and/or salts thereof (for example, the sodium salt) and/or homologues, analogues derivatives, complexes, esters, fragments, and/or sub-units of hyaluronic acid (preferably hyaluronic acid and salts thereof) to transport (facilitate or cause the transport of) the drug (for example NSAID) into the skin or exposed tissue to the site of the disease or condition to be treated percutaneously, (to the site of trauma and/or pathology), for example into the epidermis, where the form of hyaluronic acid and medicine accumulates and remains for a prolonged period of time thereby for example blocking prostaglandin synthesis in the skin or exposed tissue. The form of hyaluronic acid is then cleared through the lymphatics (lymphatics system).

The use of the composition, formulation or combination for the treatment of the diseases and conditions aforesaid may be for example by applying dosage amounts of the composition, formulation or combination a number of times daily (for example, 3 times daily) for a period of time, for example, 2-4 weeks to clear the disease, lesion or condition. Each dosage amount applied will depend upon the size of the lesion or condition on the skin or exposed tissue. The dosage amount include 5-10 mg of the form of hyaluronic acid per 1 cm² skin area or exposed tissue area.

One such formulation may comprise 3% (by weight) diclofenac in a 2½% (by weight) hyaluronic acid (sodium hyaluronate - molecular weight 661,600) gel formulation, with the excipients being glycerine (5%), benzyl alcohol (3%) (acting in part as a solubilizer and preservative), and sterile water (the balance) in a 50 gm tube of the composition (a plurality of dosage amounts) whose tube O.D. (outer diameter) of the opening through which the gel formulation is discharged from the tube is 8 mm and whose I.D. (inner diameter) of the opening is 4 mm. Therefore a ribbon 2-3 cm in length, squeezed from a tube gives about 5mg-7½ mg of hyaluronic acid for application to a skin or exposed tissue surface area of 1-1½ cm² with an effective dosage amount of diclofenac. While greater amounts squeezed from the tube may be applied, the application of substantial excessive dosage amounts to the skin and/or exposed tissue may saturate the skin or exposed tissue and thus the epidermis. (There is therefore no more room for the composition to pass between the cells and therefore further applications at that time will not provide additional benefit.)

Another formulation may comprise 3% (by weight) diclofenac in a 2½% (by weight) hyaluronic acid (sodium hyaluronate - molecular weight 679,000) gel formulation (also in a tube) with excipients being benzyl alcohol (1%) (a preservative), methoxypolyethylene glycol 350 (20% by weight) (a solubilizer), and sterile water (the balance).

While the above compositions, combinations and formulations are proposed, provided there is sufficient amounts of the form of hyaluronic acid (for example, sodium hyaluronate) in the dosage amounts applied to the skin and/or exposed tissue to facilitate or cause the percutaneous (intracutaneous) transport of the drug for example which inhibits prostaglandin synthesis, preferably an NSAID (for example, diclofenac) to block prostaglandin synthesis, then the formulations may be of any suitable form, for example, a 1% lotion of hyaluronic acid with NSAID, or a cream or gel or any other suitable form.

Containers (for example tubes and jars) may be provided containing compositions comprising a plurality of dosage amounts of the drug and form of hyaluronic acid, each dosage amount comprising an effective non-toxic dosage amount of the drug and an effective non-toxic dosage amount of the form of hyaluronic acid (preferably sodium hyaluronate having molecular weight less than about 750,000 daltons) to transport the drug into the skin and/or exposed tissue. In some embodiments, means are provided to assist the removal from the container of an effective dosage amount of the composition in the container for use to apply to the skin or exposed tissue at the site of trauma and/or pathology to treat the disease and/or condition (for example mouth opening of a tube to control the amount discharged from the tube).

Furthermore, because there is little concern with respect to the toxicity or adverse effects of the use of, for example, the NSAIDs with the hyaluronic acid in the compositions of this invention the NSAID may be combined as needed (after solubilizing (if required) of the NSAID in a suitable solubilizer) with the form of the hyaluronic acid.

Applicants postulate that the hyaluronic acid and/or salts thereof and/or homologues, analogues, derivatives, complexes, esters, fragments, and/or subunits of hyaluronic acid facilitate or cause the transport of the drug for example which blocks prostaglandin synthesis (preferably an NSAID) to the site of prostaglandin synthesis to block prostaglandin synthesis.

Applicant's compositions and dosage amounts of their compositions and the use of their compositions and dosage amounts of their compositions, at the same time, abate pain that the patient is experiencing at the paccinian nerve bundles (superficial nerve bundles) at the site of the trauma and/or pathology on/in the exposed tissue and/or skin.

The combination of hyaluronic acid and salts thereof and other forms with drugs for example that inhibit prostaglandin synthesis, for example NSAIDs, alters their distribution and performance in the skin and/or exposed tissue particularly the epidermis (the combinations and formulations being systemic independent), and produces an unusual targeting for underperfused skin and/or pathological tissue in the skin (site of trauma and/or pathology).

As a major amount of soluble indomethacin may be incorporated into the topical formulation, or composition, the indomethacin may be solubilized using n-methyl glucamine at a dilution of 5mg/ml of n-methyl glucamine (NMG). This substance is then passed through a 22 micron Milipore filter to produce sterility. This material is non-toxic at 16 fold the therapeutic dose in animals (with hyaluronic acid) and for this reason was considered appropriate to be used in human conditions. Thus, Indocid™ solubilized in NMG may be administered with hyaluronic acid topically for percutaneous penetration at, for example, varying doses. The solution of indomethacin and NMG may be mixed with, for example, "LifeCore™" hyaluronic acid in dosage amounts discussed above. This produces an appropriate mixture and can be administered safely.

When the NSAID, for example indomethacin (dissolved in n-methyl glucamine) or other NSAID, is applied topically in an effective dosage amount from a composition or formulation also including the effective dosage amount of the form of hyaluronic acid, no major toxic side effects occur, such as gastro-intestinal distress, neurological abnormalities, depression, etc., even at elevated amounts of indomethacin (if necessary). (This may be in part because of the clearing of the hyaluronic acid through the lymphatic system from the site). In addition, the responses that have been observed are dramatic when the drug for example NSAID (for example diclofenac) is combined with hyaluronic acid, demonstrating clearly that the combination is now "targeting" to the site of pathology or trauma, or pathological tissue. Furthermore, patients using the formulations and combinations of drug (for example NSAID) - hyaluronic acid (sodium hyaluronate) (for example, diclofenac or indomethacin and hyaluronic acid), experience dramatic relief of pain immediately.

Thus, Applicants believe that the use of the NSAID, for example with hyaluronic acid (sodium hyaluronate), deblocks the macrophages (and N.K. cells (Natural Killer Cells) thought to be immature macrophages) by preventing enzymatic production of prostaglandin which blocks macrophage (and N.K cell) functioning. The hyaluronic acid (and salt and other forms) not only enhances the activity of the drug (NSAID) but also reduces any side effects and toxicity that is associated with the use of the prostaglandin synthesis inhibitors. When effective dosage amounts of compositions, formulations and combinations containing effective dosage amounts of the drugs for example, (NSAIDs (for example, diclofenac)) and effective dosage amounts of, for example, hyaluronic acid or the sodium salt thereof, are applied to for example the tumour lesion (for example basal cell carcinoma) or other condition (for example, actinic keratoses lesion) for a period of time (for example, 3 times daily for 2-4 weeks), the carcinoma and lesions, as the case may be, disappear.

Applicants also postulate that when the combination or formulation is applied to the disease or condition (for example, basal cell carcinoma or actinic keratoses), the hyaluronic acid passes between the cells (in the stratum corneum and epidermis to the dermis depending on amounts) to the areas of trauma and/or pathology deficient in hyaluronic acid (or forms thereof), transporting, taking drawing, carrying or pulling the NSAID with it to the sites of prostaglandin synthesis, penetrating to inhibit prostaglandin synthesis until the space between the cells is saturated. The NSAID now being proximate the Paccinian nerve bundle (superficial nerve bundles at the end of the nerves) gives pain relief. The macrophages (which had been previously blocked) are unblocked and act to destroy the disease or condition for example basal cell carcinoma, actinic keratoses lesion, or other disease or lesion. Furthermore, the effective non-toxic dosage amount of the composition, combination or formulation, comprising the effective dosage amount of the form of hyaluronic acid and the effective dosage amount of NSAID passing through the stratum corneum to the epidermis and to the dermis (if a sufficient amount of the form of hyaluronic acid is present), passes into the skin, accumulating and staying longer in the skin at the site of the trauma and/or pathology. Therefore, after having had an immediate effect at the site of trauma and/or pathology (for example, relieving pain and acting on the basal cell carcinoma, actinic keratoses and other disease, condition or lesion), the NSAID-hyaluronic acid combination continues to accumulate at the site in need of treatment and thereafter clears through the lymphatic system.

Fifteen (15) minutes after application of one of Applicants' formulations, about three times the amount of Applicants' formulation has penetrated into the skin (particularly the epidermis) than formulations and combinations not containing the effective dosage amounts of hyaluronic acid, but containing the same drug. Furthermore, the drug and hyaluronic acid accumulate and remain at the site in need of treatment for a longer period of time.
The amount of the form of hyaluronic acid in each dosage amount administered is greater than 5-10 mg/cm² and the molecular weight is less than about 750,000 daltons.

We have compared the penetration and retention of one of our combinations (formulations) with a control and Voltarol Emulgel in the skin as follows:

| (A) OUR FORMULATION | | | | |
|---|---|---|---|---|
| 1% DICLOFENAC IN 3.0% HA GEL 50g/tube EPDICLO1 LOT XPB 044 Quantity 1500ml | | | | |
| FORMULA | Supplier | Lot | Amount | Percent |
| Sterile Water | Baxter | AW45F1 | 1397ml | -- |
| Glycerin | Life | 1043 | 45g(36ml) | 3% |
| Benzyl Alcohol | Caledon | 02517 | 22.5g(22ml) | 1.5% |
| Liquid Wax DICDD | Brooks | 191-175 | 45g | 3% |
| Diclofenac Sodium | Prosintex | 9113003 | 15g | 1% |
| Sodium Hyaluronate | Skymart | HG-1103 | 45g | 3% |
| Mol. Wt. 661,600 | | | | |

### PROCEDURE

- Set up stirring apparatus using a 3 liter stainless steel beaker
- Add Water, Glycerin, Benzyl Alcohol and Liquid Wax DICDD, stir and mix for 10 minutes
- Add Diclofenac Sodium and stir for 30 minutes to dissolve
- Add Sodium Hyaluronate and stir for 90 minutes

### FILLED

In a 50 ml aluminum collapsible tube,
inside of tube lacquered with a phanolic resin, outside of tube white regular enamel coating;
9 mm white polypropylene screw on cap with pierce tip

| | Gels | Batch No.s |
|---|---|---|
| (B) | Voltarol Emulgel | 060400 10 93 |
| (C) | 1% Diclofenac Gel | XPBO49 (Control) |

| (C) CONTROL | | | | |
|---|---|---|---|---|
| 1% DICTOFENAC IN CARAPOL GEL, 50g Jar LOT XPB 049 Quantity 100ml | | | | |
| FORMULA | Supplier | Lot | Amount | Percent |
| Sterile Water | Baxter | AW45N5 | 93ml | -- |
| Glycerin | BDH | 2579 | 3g | 3% |
| Benzyl Alcohol | BDH | 23797 | 1.5g | 1.5% |
| Liquid Wax DICDD | Brooks | L-1424 | 3g | 3% |
| Diclofenac Sodium | Prosintex | 9113003 | 1g | 1% |
| Carbopol 934 | A&C Chemicals | 910304 | 1g | 1% |

### PROCEDURE

- Set up stirring apparatus using a 400ml stainless steel beaker
- Add Water, Glycerin, Benzyl Alcohol, Liquid Wax DICDD, and stir to mix thoroughly for 10 minutes
- Add Diclofenac Sodium and stir for 20 minutes to dissolve
- Very slowly add Carbopol 934, avoid getting lumps

### Samples

| Cell | Sample | Quantity of gel applied (mg) |
|---|---|---|
| A | 060400 10 93 | 192 |
| B | 060400 10 93 | 192 |
| C | EPDICLO1* | 192 |
| D | EPDICLO1* | 192 |
| E | XPB049 | 192 |
| F | XPB049 | 192 |

| | | |
|---|---|---|
| * - Our Formulation | | |

### Skin Type

One piece of skin (Female, 37 years, smoker, breast skin) was used for one sample from each batch. A second piece of skin (no further details available) was used for the second sample from each batch. The skin was stored deep frozen (<-20°C) until thawed for this experiment. Full thickness skin was used for this experiment.

### Experimental Conditions

Skin permeation cells were prepared containing an exposed skin surface area of 9.6 cm² and a constantly stirred receptor fluid beneath the skin consisting of 135 ml of ethanol:phosphate buffered saline (25:75 v/v).

Each cell was allowed to equilibrate for 1 hour at 37°C after which the gel was spread evenly over the skin surface at a concentration of 20 mg/cm²). See table above. The cell was then maintained at 37°C with an air temperature above the skin of 35°C.

24 hours after application of the gel the experiment was stopped and a portion of the receptor fluid removed. The skin was removed from the cell and any gel remaining on the surface carefully wiped off with dry paper towel followed by paper towel moistened with water. The skin was cut with a scalpel to obtain thin top and thicker lower sections of skin.

This was done in order to obtain layers of skin which approximated the epidermal and dermal layers. Each skin section was weighed and the residual diclofenac extracted with 10ml of fresh receptor fluid using an ultra turrax homogeniser. The homogenates were centrifuged and a portion of the resultant supernatant solutions removed.

The receptor fluid and skin extracts from each cell were assayed for diclofenac content by using a validated reverse phase high performance liquid chromatography (HPLC) method.

### Results

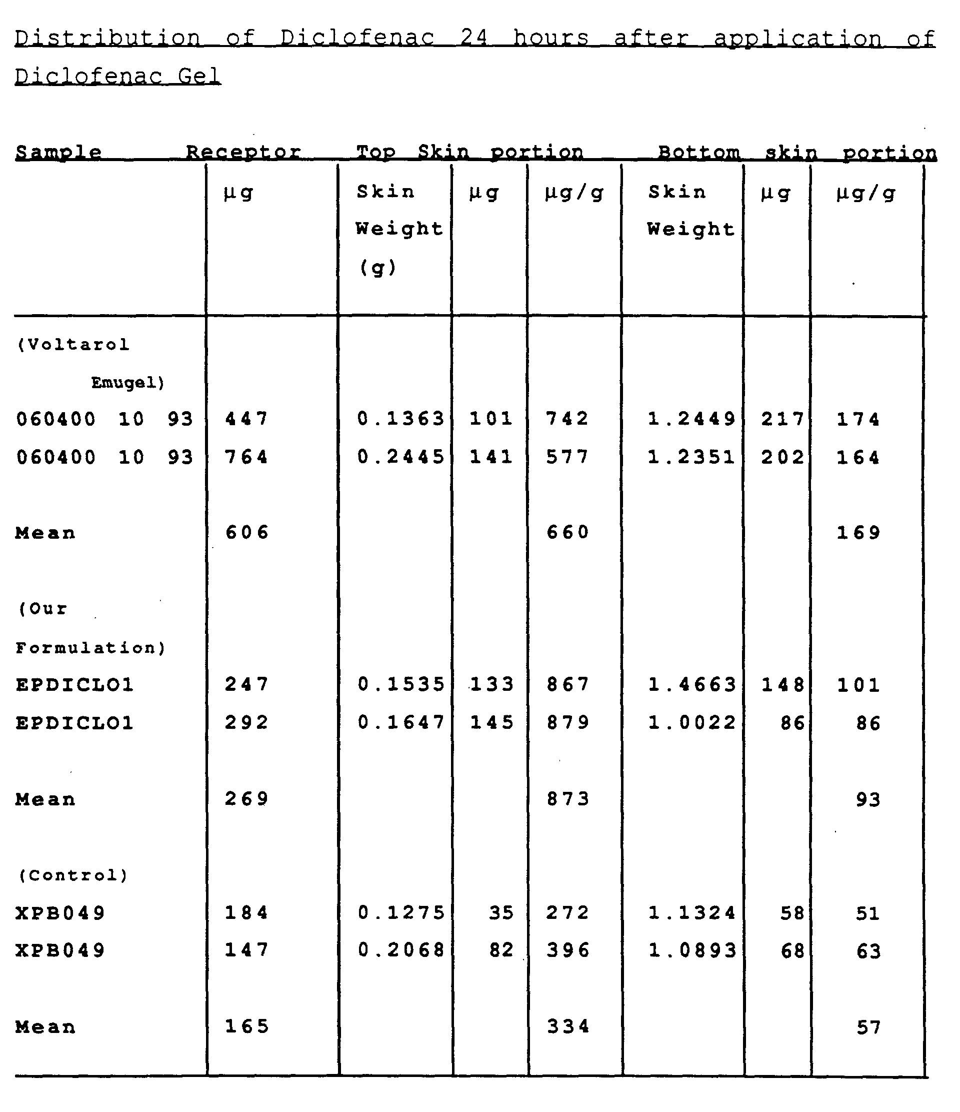

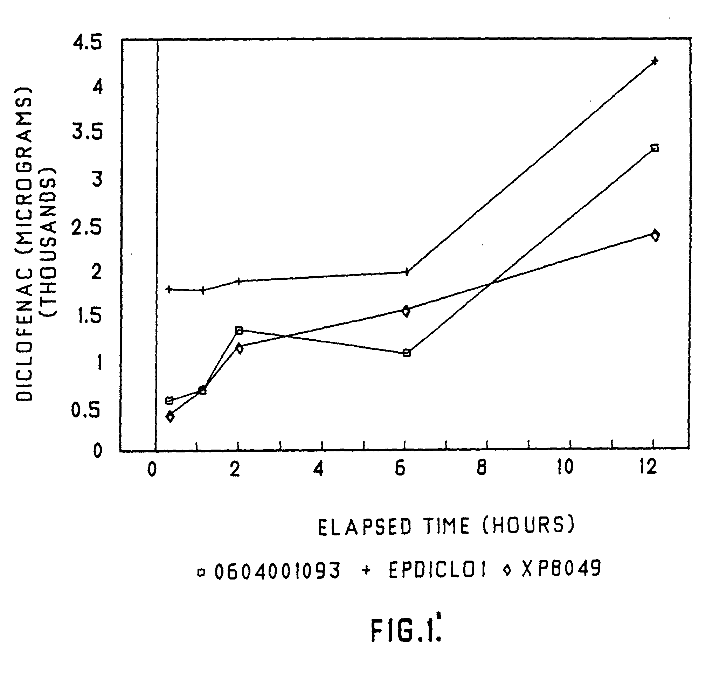

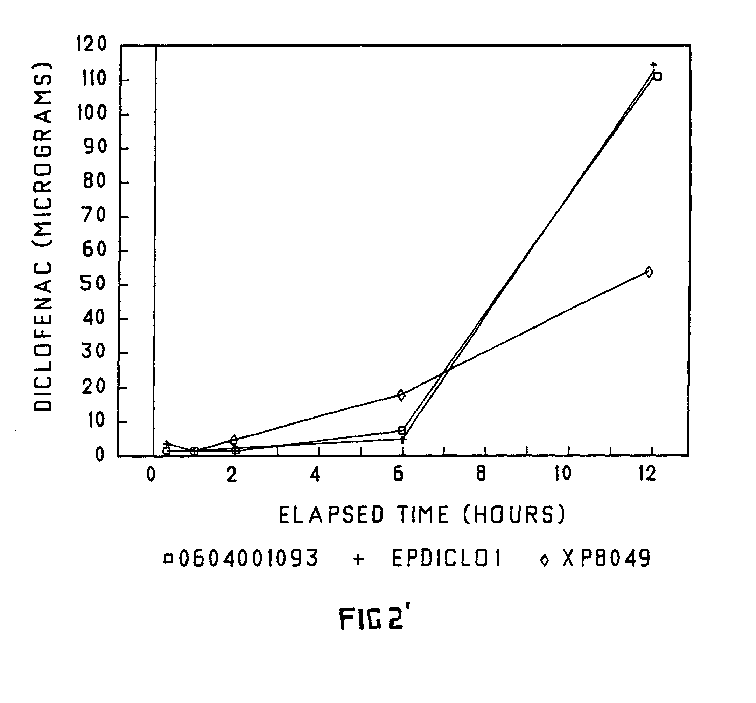

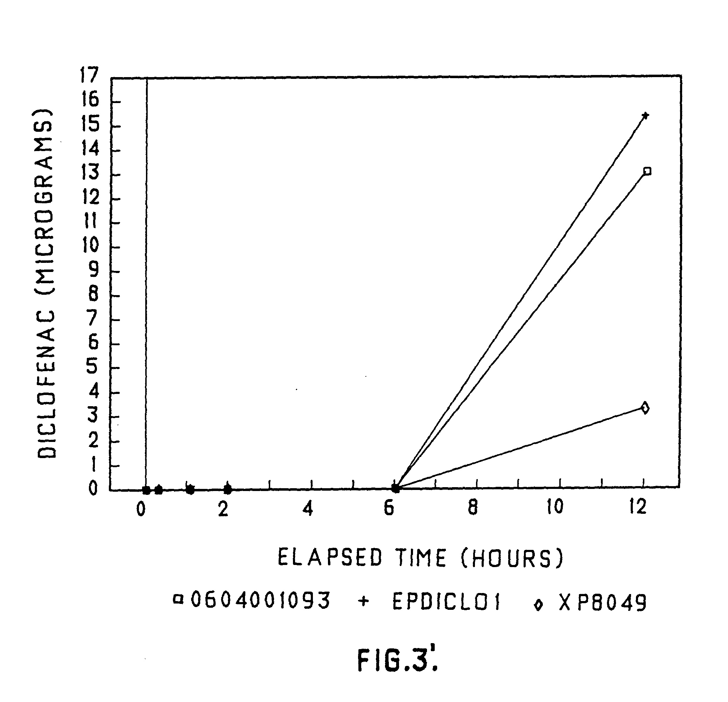

Thus having regard to the above and Figures 1', 2' and 3', it is clear that the sodium hyaluronate takes the diclofenac into the skin to the epidermis level (See Figure 1') more rapidly than the Voltarol Emugel or non-hyaluronic acid diclofenac containing control formulation, accumulates it there and retains it there longer. The other formulations permit the NSAID, diclofenac, to pass through the bottom skin portion (dermis) quicker, thereby clearing it from the epidermis and dermis, quicker. Furthermore, more of Applicants' formulation is in the epidermis and in the dermis even after 12 hours. With respect to Figure 1', the top of the graph should have the following heading "DICLOFENAC TOP SKIN PORTION", the left side of the graph should have the following side heading "DICLOFENAC (MICROGRAMS) (THOUSANDS)" and the bottom of the graph should have the following bottom heading "ELAPSED TIME (HOURS) □ 0604001093 + EPDICLO1 ◇ XP8049". With respect to Figure 2', the top of the graph should have the following heading "DICLOFENAC BOTTOM SKIN PORTION", the left side of the graph should have the following side heading "DICLOFENAC (MICROGRAMS)" and the bottom of the graph should have the following bottom heading "ELAPSED TIME (HOURS) □ 0604001093 + EPDICLO1 ◇ XP8049". With respect to Figure 3', the top of the graph should have the following heading "DICLOFENAC RECEPTOR SOLUTION", the left side of the graph should have the following side heading "DICLOFENAC (MICROGRAMS)" and the bottom of the graph should have the following bottom heading "ELAPSED TIME (HOURS) □ 0604001093 + EPDICL01 ◇ XP8049".

It is also clear that Applicants' formulations clear into the lymphatic system not through the blood system. Yet the prior art topical formulations have always tried "to drive" the formulations through the skin into the blood for treatment of the disease or condition in the area (i.e. systemic action).

Thus, our composition, formulation and combination, (and dosage amounts thereof) penetrate quickly and rapidly at the site of treatment through the upper skin into the epidermis, where the paccinian bundles are located and the NSAID and the form of hyaluronic acid are accumulated and are retained longer, where needed (for example for the treatment of basal cell carcinoma).

Further, the NSAIDs are retained in the area to be treated with the form of hyaluronic acid. In doing so, they preclude prostaglandin synthesis , in effect, deactivating the synthesis or inhibiting the synthesis, of prostaglandins, permitting the macrophages' scavenger cell activity to eliminate the tumour and lesion. Additionally, a rapid onset of pain relief (analgesic effect) is provided (depending on the amount of NSAID and form of hyaluronic acid) usually where in excess of about 10 mg of the form of hyaluronic acid (preferably hyaluronic acid and salts thereof) is administered per cm² of surface area comprises the dosage amount administered. However, there are no blood levels of the NSAID in the immediate area of treatment. The forms of hyaluronic acid are thus cleared via the lymphatic system. Then the lymphatics pass the forms of hyaluronic acid, Applicants believe, to the blood system. Thus, the NSAIDs and forms of hyaluronic acid stay at the site to be treated for well in excess of 12 - 24 hours, a protracted stay.

Thus, over the period of treatment (for example, applications of effective non-toxic dosage amounts of compositions containing for example effective non-toxic dosage amounts of the NSAIDS and effective non-toxic dosage amounts of the sodium hyaluronate, 3 times a day for 2-4 weeks, transport the NSAIDS to to the epidermis to inhibit prostaglandin synthesis to enable the macrophages to "scavenge" the tumour cells and eliminate them. The end result is the successful treatment of the disease or condition at the site of trauma and/or pathology of the skin or exposed tissue, for example, the resolution of, the basal cell carcinoma, the precancerous, often recurrent, actinic keratoses lesions, fungal lesions, "liver" spots and like lesions (found for the most part in the epidermis), squamous cell tumours, metastatic cancer of the breast to the skin, malignancies and/or tumours in the skin, primary and metastatic melanoma in the skin, genital warts cervical cancer, and HPV (Human Papilloma Virus) including HPV of the cervix, psoriasis (both plaque-type psoriasis and nail bed psoriasis), corns on the feet and hair loss on the head of pregnant women, with complete disappearance of the disease or condition as the case may be, by topical therapy without resorting to surgery.

One of the formulations which we have employed successfully is a gel formulation comprising 3% diclofenac in 2.5% sodium hyaluronate formulated as follows:

### Formulation 1 (3000 ml.)

| Formula | Supplier | (LOT) | Amount | Percent |
|---|---|---|---|---|
| Glycerine | Life | 1043 | 150 g (119 ml) | 5 |
| Benzyl Alcohol | Caledon | 02517 | 90 g (86 ml) | 3 |
| Diclofenac Sodium | Prosintex | 9113003 | 90 grams | 3 |
| Sodium Hyaluronate (MW 661,660) | Skymark | HG1003 | 75 grams | 2.5 |
| Sterile water balance | Baxter | AW4455 | 2795 ml. | |

### Procedure

- set up stirring apparatus using a 4 litre stainless steel beaker
- add water, Glycerine, and Benzyl Alcohol; stir to mix
- add Diclofenac Sodium and stir for 30 minutes
- then add the Sodium Hyaluronate and stir for 90 minutes
- initially, stir at a high torque but avoid splashing; as the gel thickens, stir at a lower torque.

The gel is then packaged in a tube or jar or other suitable container for use. Identification of suitable dosage amounts and how they are taken from the container may be provided with the container - for example squeeze "X" cm. of ribbon from the tube; fill spoon or spatula accompanying jar; (the spoon or spatula containing a predetermined dosage amount) then apply and rub into site of trauma and/or pathology (the dosage amount indicated will be such amount of the composition which comprises in excess of about 5 mg. of sodium hyaluronate per cm² (square centimeter) of skin or exposed tissue to which the dosage amount is to be applied. The amount of Diclofenac Sodium was determined in the same manner (having regard to the dosage amount required).

Another such formulation is:

### Formulation 2

| Formula | Supplier | (LOT) | Amount | Percent |
|---|---|---|---|---|
| Methoxypolyethylene Glycol 350 | Sigma | 34F-0266 | 300 g. | 20 |
| Benzyl Alcohol | BDH | 23797 | 15 g. | 1 |
| Diclofenac Sodium | Prosintex | 9123012 | 45 g. | 3 |
| Sodium Hyaluronate (MW 679,000) | Skymart | HG 1004 | 37.5 g. | 2.5 |
| Sterile Water balance | Baxter | AW45R6 | 1200 ml. | |

### Procedure

- set up stirring apparatus using a 3 litre stainless steel beaker
- add water, Methoxypolyethylene Glycol 350, and Benzyl Alcohol and stir for 20 minutes to mix
- add Diclofenac Sodium and stir for 30 minutes to dissolve
- add Hyaluronate Sodium slowly and stir initially at a high speed, but avoid splashing
- after addition, stir at a slower speed for 90 minutes; the slower speed reduces the formation of air bubbles
- the result is a clear, transparent, viscous gel which is put into a container. Once again instructions are given for administration and if applicable measuring devices (to provide a premeasured dosage amount) accompany the container.
Still other formulations are:

### Formulation 3

| 3% Diclofenac in 2.5% HA Gel | | | | |
|---|---|---|---|---|
| Formula | Supplier | LOT | Amount | Percent |
| Sterile Water | Baxter | AW45K6 | 1200 ml | - |
| Methoxypolyethylene Glycol 350 | Sigma | 34F-0266 | 300G (273 ml) | 20% |
| Benzyl Alcohol | BDH | 23797 | 15G (14 ml) | 1% |
| Diclofenac Sodium | Prosintex | 9123012 | 45 g | 3% |
| Sodium Hyaluronate MW 679,000 | Skymart | HG 1004 | 37.5 g | 2.5% |

### Procedure

- Set up stirring apparatus using a 2 liter stainless steel beaker,
- Add water, Methoxypolyethylene Glycol 350, and Benzyl Alcohol and stir for 20 minutes to mix,
- Add Diclofenoc Sodium and stir for 30 minutes to disolve,
- Add Hyularonate Sodium slowly and stir initially at a high speed, but avoid splashing,
- After addition, stir at a slower speed for 90 minutes,the slower speed reduces the formation of air bubbles,
- The result is a clear transparent, viscous gel which is poured into jars and tubes. Once again instructions accompany the container and where applicable appropriate devices for providing a premeasured amount of the composition accompany the container.

### Formulation 4

| 5% IBUPROFEN IN 3.0% HA GEL, 50 ml JAR | | | | |
|---|---|---|---|---|
| Formula | Supplier | LOT | Amount | Percent |
| Sterile Water | Baxter | AW45R6 | 196 ml | -- |
| Meglumine | Falk | 15684 | 11 g | 5.5% |
| Ibuprofen | BDH | 19/241 | 10 g | 5% |
| Benzy Alcohol | BDH | 23797 | 2 g | 1% |
| Glycerin | BDH | 2579 | 2 g | 1% |
| Hyaluronate Sodium Mol Wt 661,600 | Skymart | HG 1003 | 6 g | 3% |

### PROCEDURE

- Set up stirring apparatus using a 300 ml stainless steel beaker,
- Add Sterile Water and Meglumine, and stir for 10 minutes,
- Add Ibuprofen and stir for 15 minutes,
- Add Benzyl Alcohol, followed by Glycerin and stir for 15 minutes,
- Finally, add Hyaluronate Sodium slowly and stir initially at a high torque to mix, but avoid splashing,
- As the gel thickens, stir at a slow speed for 90 minutes.

### Formulation 5

| 2% PIROXICAM IN 2.5% HA GEL | | | | |
|---|---|---|---|---|
| Formula | Supplier | LOT | Amount | Percent |
| Sterile Water | Baxter | AW45R6 | 200 ml | -- |
| Meglumine | Falk | 15684 | 8 g | 4% |
| Piroxicam | AMSA | 1-010 | 4 g | 2% |
| Hyaluronate Sodium MW 661,600 | Skymart | HG 1003 | 5 g | 2.5% |

### PROCEDURE

- Set up stirring apparatus using a 300 ml stainless steel beaker,
- Add 200 ml of sterile water,
- Add 8 grams of Meglumine and dissolve,
- Very slowly add 4 grams of Piroxicam and stir for 20 minutes,
- Slowly add 5 grams of Hyaluronate Sodium and stir at high speed,
- Stir for 90 minutes at a slower speed

### COMMENTS

- A clear yellowish transparent gel

### Formulation 6

| 5% IBUPROFEN CREAM, 50 ml JAR | | | | |
|---|---|---|---|---|
| OILY PHASE | | | | |
| Formula | Supplier | LOT | Amount | Percent |
| Liquid wax DICDD | Brooks | L-1424 | 450 g | 15% |
| Brookswax D | Brooks | P-490 | 480 g | 16% |
| Glycerin | BDH | 109109/2578 | 150 g (119 ml) | 5% |

| AQUEOUS PHASE | | | | |
|---|---|---|---|---|
| Sterile Water | Baxter | AW45F1 | 1950 ml | -- |
| Meglumine | Falk | 15684 | 150 g | 5% |
| Ibuprofen | BKH | 19/241 | 150 g | 5% |
| MW 200,00 | | | | |
| Sodium Hyaluronate | Skymart | O01 | 45 g | 1.5% |
| Preservative Suttocide | A Sutton | SH-107 | 9 | 0.3% |

### PROCEDURE

A - Add all the ingredients of the oily phase A into a 4 liter stainless steel beaker, melt at 55°c, finally heat to 75% when Aqueous Phase B is ready
B - Into a 3 liter stainless steel beaker, add 1950 ml water, set up, the stirring apparatus, add the Meglumine, stir to dissolve for 10 minutes,
   - Slowly add Ibuprofen, stir to dissolve for 20 minutes,
   - Very slowly add Sodium Hyaluronate and stir for one hour to dissolve all the Sodium Hyaluronate,
   - Finally, heat to 75°C, with stirring for a total time of 30 minutes.

### POUR B INTO A, both at a temperature of 75°C, slowly

- Remove the heat source and stir with a strong vortex for one hour,
- When the temperature has cooled down to 45°C add preservative Suttocide A,
- Continue stirring at a slower speed until the temperature is 35°C,
- At 35°C remove the propeller, pour into 50 ml jars.

### Formulation 7

| 1% DICLOFENAC IN 3% HA Gel, 50 ml jar Quantity 3000ml | | | | |
|---|---|---|---|---|
| Formula | Supplier | LOT | Amount | Percent |
| Sterile Water | Baxter | AW45R6 | 2796ml | -% |
| Glycerin | BDH | 2579 | 50g(71ml) | 3% |
| Benzyl Alcohol | BDH | 23797 | 45g(43ml) | 1.5% |
| Liquid wax DICDD | Brooks | 191-175 | 90 g | 3% |
| Diclofenac Sodium | Prosintex | 9113003 | 30 g | 1% |
| Hyaluronate Sodium MW 679,000 | Skymout | HG 1004 | 90 g | 3% |

### PROCEDURE

- Set up stirring apparatus using a 4 liter stainless steel beaker.
- Add water, Glycerin, Benzyl Alcohol and Liquid wax DICDD and stir to mix thoroughly for 10 minutes
- Add Diclofenac Sodium and stir for 30 minutes to dissolve.
- Slowly add Hyaluronate Sodium, stirring at a high torque initially during addition.
- After addition stir at a slower speed for 90 minutes.
- A white opaque viscous gel is formed.

### Formulation 8

| 1% DICLOFENAC IN 3.0% HA Gel, 50 ml tube Quantity 1500 ml | | | | |
|---|---|---|---|---|
| Formula | Supplier | TOT | Amount | Percent |
| Sterile Water | Baxter | AW45F1 | 1397 ml | -% |
| Glycerin | Life | 1043 | 45g(36 ml) | 3% |
| Benzyl Alcohol | Caledon | 02517 | 22.5g(22ml) | 1.5% |
| Liquid wax DICDD | Brooks | 191-175 | 45 g | 3% |
| Diclofenac Sodium | Prosintex | 9113003 | 15 g | 1% |
| Sodium Hyaluronate Mol. Wt. 661,600 | Skymart | HG 1003 | 45 g | 3% |

### PROCEDURE

- Set up stirring apparatus using a 3 liter stainless steel beaker.
- Add water, Glycerin, Benzyl Alcohol and Liquiwax DICDD, stir to mix for 10 minutes.
- Add Diclofenac Sodium and stir for 30 minutes to dissolve.
- Add Sodium Hyaluronate and stir for 90 minutes.

### Formulation 9

| HYANALGESE CREAM (L) 50 ml tube Quantity 3000 ml | | | | |
|---|---|---|---|---|
| FORMULA | | | | |
| A. Oily Phase | SUPPLIER | LOT | AMOUNT | PERCENT |
| Liquid Wax DICDD | Brooks/Amisol | | 450g | 15.0% |
| Brookswax D | Brooks/Amisol | | 480g | 16.0% |
| Glycerine | Amisol | | 150g | 5.0% |

| B. Aqueous Phase | | | | |
|---|---|---|---|---|
| Sterile Water | Baxter | AW4YA8 | 1950ml | -% |
| Meglumine | Falk | | 150g | 5.0% |
| Sodium Hyaluronate MW 207,000 | Skymart | PO1 | 45g | 1.5% |
| Ibuprofen | BDH | | 150g | 5.0% |
| Suttocide A | Sutton | | 9.0g | 0.3% |

### PROCEDURE

A. - Add all the ingredients of the oily phase into a 4 liter stainless steel beaker, melt at 55°C, finally heat to 75°C when aqueous phase is ready (at 75°C) to pour in.
B. - Into another 4 liter stainless steel beaker, add 1950 ml water.
   - Set up the stirring apparatus and add the Meglumine
   - Stir to dissolve with high torque, then slowly add Ibuprofen
   - When the Ibuprofen is dissolved, slowly add Sodium Hyaluronate
   - Stir cold for one hour to dissolve all the ingredients
   - Finally heat to 75°C and stir roughly throughout a 30 minute period

### MIX B INTO A

- Slowly pour B into A (both at 75°C) with stirring
- Immediately remove the hot plate (heat) and stir
- Stir with a strong vortex for one hour
- When the temperature is 45°C, add the preservative Suttocide A
- Stir for about an hour to cool to 35°C
- At 35°C remove the propeller and pour into 50 ml tubes
- Pour 50 grams of the cream into each tube

| 1% BANAMINE IN 2.5% HA GEL (L) XPB 041 Quantity 3000 ml | | | | |
|---|---|---|---|---|
| FORMULA | | | | |
| | SUPPLIER | LOT | AMOUNT | PERCENT |
| Sterile Water | Boxter | AW4SA2 | 2400 m | 1--% |
| Sodium Hyaluronite MW 661,600 | Skymart | HE1003 | 75g | 2.5% |
| *Banamine, 100 ml vial | Scheing | ○ CNXB13 | 300 ml | 1% |
| Banamine, 100 ml vial | Scheing | ○ CNXB12 | 300 ml | 1% |
| | | | 3000 ml | |

| | | | | |
|---|---|---|---|---|
| (50 mg/ml) 600 = 30,000mg = 30 grams Flunixin in 600 ml *Banamine contains Flunixin Meglumine (50 mg Flunixin per ml) or 83 mg Flunixin Meglumine | | | | |

### PROCEDURE

- Set up stirring apparatus using a 4 liter stainless steel beaker
- Add water, stir with a strong vortex, then add sodium Hyoluronate slowly
- Then immediately add the Banamine, stir the mixture for 4 hours.

One form of hyaluronic acid and/or salts thereof (for example sodium salt) and homologues, analogues, derivatives, complexes, esters, fragments, and sub-units of hyaluronic acid, preferably hyaluronic acid and salts and thereof, suitable for use with Applicant's invention is a fraction supplied by Hyal Pharmaceuticals Limited. One such fraction is a 15 ml vial of Sodium hyaluronate 20mg/ml (300mg/vial - Lot 2F3). The sodium hyaluronate fraction is a 2% solution with a mean average molecular weight of about 225,000. The fraction also contains water q.s. which is triple distilled and sterile in accordance with the U.S.P. for injection formulations. The vials of hyaluronic acid and/or salts thereof may be carried in a Type 1 borosilicate glass vial closed by a butyl stopper which does not react with the contents of the vial.

The fraction of hyaluronic acid and/or salts thereof (for example sodium salt) and homologues, analogues, derivatives, complexes, esters, fragments, and sub-units of hyaluronic acid, preferably hyaluronic acid and salts thereof, may comprise hyaluronic acid and/or salts thereof having the following characteristics:
a purified, substantially pyrogen-free fraction of hyaluronic acid obtained from a natural source having at least one characteristic selected from the group (and preferably all characteristics) consisting of the following:
   i) a molecular weight within the range of 150,000-225,000;
   ii) less than about 1.25% sulphated mucopolysaccharides on a total weight basis;
   iii) less than about 0.6% protein on a total weight basis;
   iv) less than about 150 ppm iron on a total weight basis;
   v) less than about 15 ppm lead on a total weight basis;
   vi) less than 0.0025% glucosamine;
   vii) less than 0.025% glucuronic acid;
   viii) less than 0.025% N-acetylglucosamine;
   ix) less than 0.0025% amino acids;
   x) a UV extinction coefficient at 257 nm of less than about 0.275;
   xi) a UV extinction coefficient at 280 nm of less than about 0.25; and
   xii) a pH within the range of 7.3-7.9.

Preferably, the hyaluronic acid is mixed with water and the fraction of hyaluronic acid has a mean average molecular weight within the range of 150,000-225,000. More preferably, the fraction of hyaluronic acid comprises at least one characteristic selected from the group (and preferably all characteristics) consisting of the following characteristics:
i) less than about 1% sulphated mucopolysaccharides on a total weight basis;
ii) less than about 0.4% protein on a total weight basis;
iii) less than about 100 ppm iron on a total weight basis;
iv) less than about 10 ppm lead on a total weight basis;
v) less than 0.00166% glucosamine;
vi) less than 0.0166% glucuronic acid;
vii) less than 0.0166% N-acetylglucosamine;
viii) less than 0.00166% amino acids;
x) a UV extinction coefficient at 257 nm of less than about 0.23;
xi) a UV extinction coefficient at 280 nm of less than 0.19; and
xii) a pH within the range of 7.5-7.7

Applicants also propose to use sodium hyaluronate produced and supplied by LifeCore™ Biomedical, Inc., having the following specifications:

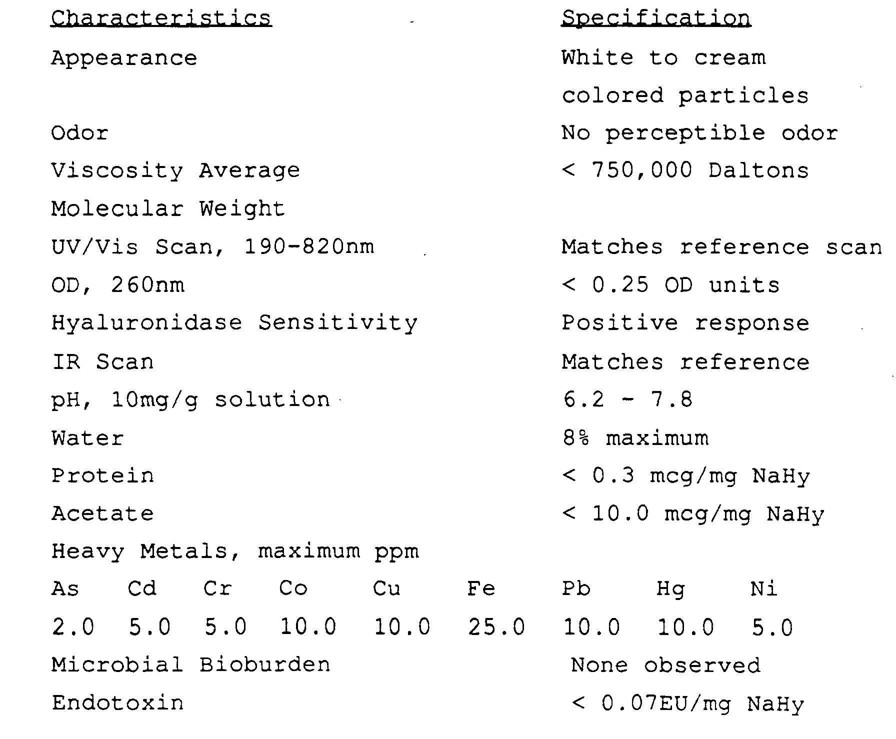

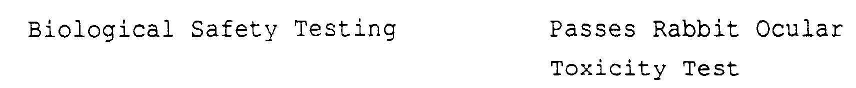

Another form of sodium hyaluronate is sold under the name Hyaluronan HA-M5070 by Skymart Enterprises, Inc. having the following specifications:

| Specifications' Test | |
|---|---|
| Results | |
| Lot No. | HG1004 |
| pH | 6.12 |
| Condroitin Sulfate | not detected |
| Protein | 0.05% |
| Heavy Metals | Not more than 20 ppm |
| Arsenic | Not more than 2 ppm |
| Loss on Drying | 2.07% |
| Residue on Ignition | 16.69% |
| Intrinsic Viscosity | 12.75 dl/s (XW: 679,000) |
| Nitrogen | 3.14% |
| Assay | 104.1% |
| Microbiological Counts | 80/g |
| E. coli | Negative |
| Mold and Yeast | Not more than 50/g |

Other forms of hyaluronic acid and/or its salts, and homologues, derivatives, complexes, esters, fragments and sub units of hyaluronic acid may be chosen from other suppliers, for example those described in prior art documents provided the form of hyaluronic acid chosen is suitable for transport of the medicine.

The following references teach hyaluronic acid, sources thereof, and processes for the manufacture and recovery thereof which may be suitable.

United States Patent 4,141,973 teaches hyaluronic acid fractions (including sodium salts) having:
"(a) an average molecular weight greater than about 750,000, preferably greater than about 1,200,000 - that is, a limiting viscosity number greater than about 1400 cm³/g., and preferably greater than about 2000 cm³/g.;
(b) a protein content of less than 0.5% by weight;
(c) ultraviolet light absorbance of a 1% solution of sodium hyaluronate of less than 3.0 at 257 nanometers wavelength and less than 2.0 at 280 nanometers wavelength;
(d) a kinematic viscosity of a 1% solution of sodium hyaluronate in physiological buffer greater than about 1000 centistokes, preferably greater than 10,000 centistokes;
(e) a molar optical rotation of a 0.1 - 0.2% sodium hyaluronate solution in physiological buffer of less than -11 X 10³ degree - cm²/mole (of disaccharide) measured at 220 nanometers;
(f) no significant cellular infiltration of the vitreous and anterior chamber, no flare in the aqueous humour, no haze or flare in the vitreous, and no pathological changes to the cornea, lens, iris, retina, and choroid of the owl monkey eye when one milliliter of a 1% solution of sodium hyaluronate dissolved in physiological buffer is implanted in the vitreous replacing approximately one-half the existing liquid vitreous, said HUA being
(g) sterile and pyrogen free and
(h) non-antigenic."

Canadian Letters Patent 1,205,031 (which refers to United States Patent 4,141,973 as prior art) refers to hyaluronic acid fractions having average molecular weights of from 50,000 to 100,000; 250,000 to 350,000; and 500,000 to 730,000 and discusses processes of their manufacture.

In order to determine the blood levels in patients using formulations made according to embodiments of the invention, a study of the pharmacokinetic profiles of two topical diclofenac formulations after repeat dosing were undertaken.

One such product was the product Voltarol Emulgel marketed in the United Kingdom by Geigy. The other was a Diclofenac preparation in Hyaluronic Acid.

This was an open, repeat dose, crossover comparison using a randomized balanced block in six healthy volunteers.

The study consisted of administration with one, two week period in between periods, each period lasting fourteen days. The test articles applied were for the first six days of each period and the seventh day was study day during which the final application is made and blood samples taken.

The approximate duration of the study including pre and post study screening was six weeks.

### Doses

Diclofenac (3.0%) with Hyaluronic Acid (2.5%)
Dose: Approximately 2 g, three times daily
Route: Topical

(W1) Voltarol Emulgel, Diclofenac diethylammonium
salt 1.16g aqueous gel (Geigy)
Dose: Approximately 2 g, three times daily
Route: Topical (W1)

### ADMINISTRATION: to suitable patients

Subjects applied one of the designated test articles topically to the calves and massaged into the skin, in a dose of approximately 2 g per application three times a day for six consecutive days. The size of a 2g dose was prepared by comparison with a silicone example given to each subject.

On the seventh day, the cream was applied once, in the same manner as before, under the supervision of the staff of the Clinical Investigation Unit.

After a washout period of one week the procedure was repeated with the alternate test article.

The following were the results of the tests:
(H = hyaluronic acid formulation)
(V = Voltarol Emulgel)

### PERIOD 1

| All concentrations ng ml - ¹ | | | | | | | | | | | | |
|---|---|---|---|---|---|---|---|---|---|---|---|---|
| SUBJECT | TIME POINT (hours) | | | | | | | | | | | |
| | 0 | 0.25 | 0.5 | 1 | 2 | 3 | 4 | 5 | 6 | 8 | 10 | 12 |
| H-1 | 10.3 | 7.1 | 6.4 | ND | ND | 5.4 | 6.5 | 5.1 | ND | ND | ND | ND |
| H-2 | ND | 5.1 | ND | 5.1 | ND | ND | ND | ND | ND | 5.1 | ND | ND |
| ND | ND | ND | 5.5 | 5.2 | ND | ND | ND | ND | ND | ND | ND | V-3 |
| ND | ND | ND | ND | ND | ND | ND | ND | ND | ND | ND | ND | H-4 |
| ND | ND | ND | ND | ND | ND | ND | ND | ND | ND | ND | ND | V-5 |
| ND | ND | ND | ND | ND | ND | ND | 8.4 | ND | ND | ND | ND | V-6 |
| ND = NONE DETECTED (>5.0 ng ml⁻¹) | | | | | | | | | | | | |

### PERIOD II

| All concentrations ng ml -1 | | | | | | | | | | | | |
|---|---|---|---|---|---|---|---|---|---|---|---|---|
| SUBJECT | TIME POINT (hours) | | | | | | | | | | | |
| | 0 | 0.25 | 0.5 | 1 | 2 | 3 | 4 | 5 | 6 | 8 | 10 | 12 |
| V-1 | ND | ND | ND | ND | ND | ND | ND | ND | ND | ND | ND | ND |
| V-2 | ND | ND | ND | ND | ND | ND | ND | ND | ND | ND | ND | ND |
| H-3 | ND | ND | ND | ND | ND | ND | ND | ND | ND | ND | ND | ND |
| V-4 | ND | ND | ND | ND | ND | ND | ND | ND | ND | ND | ND | ND |
| H-5 | ND | ND | ND | ND | ND | ND | ND | ND | ND | ND | ND | ND |
| H-6 | ND | ND | ND | ND | ND | ND | ND | ND | ND | ND | ND | ND |
| ND = NONE DETECTED (>5.0 ng ml⁻¹) | | | | | | | | | | | | |

Other tests were undertaken to determine blood levels comparing Proflex (a formulation containing Ibuprofen) and the following formulation containing hyaluronic acid and Ibuprofen.

| HYANALGESE CREAM (L) X PB 022 - 50 ml tube Quantity 3000 ml | | | | |
|---|---|---|---|---|
| FORMULA | | | | |
| A. Oily Phase | SUPPLIER | LOT | AMOUNT PERCENT | |
| Liquid Wax DICDD | Brooks/Amisol | | 450g | 15.0% |
| Brookswax D | Brooks/Amisol | | 480g | 16.0% |
| Glycerine | Amisol | | 150g | 5.0% |

| B. Aqueous Phase | | | | |
|---|---|---|---|---|
| Sterile Water | Baxter | AW4YA8 | 1950ml | -% |
| Meglumine | Falk | | 150g | 5.0% |
| Sodium Hyaluronate MW 207,000 | Skymart | PO1 | 45g | 1.5% |
| Ibuprofen | BDH | | 150g | 5.0% |
| Suttocide A | Sutton | | 9.0g | 0.3% |

The following were the results

| (A) PROFLEX | | | | | | | | | | | | | |
|---|---|---|---|---|---|---|---|---|---|---|---|---|---|
| SUBJECT Number | | Time after administration (Hours) | | | | | | | | | | | |
| | PD | 0 | 0.25 | 0.5 | 1 | 2 | 3 | 4 | 5 | 6 | 8 | 10 | 12 |
| 1 | ND | 0.41 | 0.37 | 0.37 | 0.32 | 0.30 | 0.27 | 0.27 | 0.24 | 0.37 | 0.31 | 0.31 | 0.16 |
| 2 | ND | 0.12 | 0.12 | 0.08 | 0.11 | 0.12 | 0.12 | 0.07 | 0.08 | 0.09 | 0.08 | ND | 0.06 |
| 3 | ND | 0.09 | 0.08 | 0.07 | ND | ND | ND | ND | ND | ND | ND | ND | ND |
| 4 | ND | 0.12 | 0.14 | 0.16 | 0.11 | 0.11 | 0.25 | 0.24 | 0.17 | 0.13 | 0.16 | 0.11 | 0.13 |
| 5 | ND | 0.14 | 0.19 | 0.19 | 0.15 | 0.16 | 0.16 | 0.14 | 0.12 | 0.11 | 0.13 | 0.10 | 0.07 |
| 6 | ND | 0.11 | 0.09 | 0.09 | 0.06 | 0.07 | 0.05 | 0.05 | 0.05 | ND | ND | ND | ND |
| Mean | 0.00 | 0.17 | 0.17 | 0.16 | 0.13 | 0.13 | 0.14 | 0.13 | 0.11 | 0.12 | 0.11 | 0.09 | 0.07 |
| S.D. | 0.00 | 0.12 | 0.10 | 0.11 | 0.10 | 0.10 | 0.10 | 0.10 | 0.08 | 0.13 | 0.11 | 0.12 | 0.06 |

| (B) HYALURONIC ACID AND IBUPROFEN | | | | | | | | | | | | | |
|---|---|---|---|---|---|---|---|---|---|---|---|---|---|
| SUBJECT Number | | Time after administration (Hours) | | | | | | | | | | | |
| | PD | 0 | 0.25 | 0.5 | 1 | 2 | 3 | 4 | 5 | 6 | 8 | 10 | 12 |
| 1 | ND | 0.11 | 0.11 | 0.12 | 0.08 | 0.08 | 0.09 | 0.11 | 0.12 | 0.08 | 0.11 | 0.16 | 0.14 |
| 2 | ND | 0.22 | 0.21 | 0.26 | 0.17 | 0.24 | 0.24 | 0.25 | 0.23 | 0.19 | 0.19 | 0.20 | 0.14 |
| 3 | ND | 0.17 | 0.10 | 0.12 | 0.09 | 0.08 | 0.07 | 0.06 | ND | 0.06 | 0.26 | 0.09 | 0.05 |
| 4 | ND | ND | ND | ND | ND | ND | ND | ND | ND | ND | ND | ND | ND |
| 5 | ND | 0.17 | 0.16 | 0.16 | 0.12 | 0.09 | 0.10 | 0.11 | 0.10 | 0.09 | 0.10 | 0.07 | ND |
| 6 | ND | 0.07 | 0.07 | 0.09 | ND | ND | ND | ND | ND | ND | ND | ND | ND |
| Mean | 0.00 | 0.12 | 0.11 | 0.13 | 0.08 | 0.08 | 0.08 | 0.09 | 0.08 | 0.07 | 0.11 | 0.09 | 0.06 |
| S.D. | 0.00 | 0.08 | 0.07 | 0.08 | 0.06 | 0.08 | 0.08 | 0.09 | 0.09 | 0.07 | 0.10 | 0.08 | 0.07 |
| ND None detected <0.05 µg/ml | | | | | | | | | | | | | |

The above clearly indicates that the blood levels are much less using hyaluronic acid to administer the NSAID.

### PRELIMINARY REPORT

A trial was conducted using a gel composition (Number 109) comprising 3% Diclofenac in 2.5% Hylauronic Acid as previously described and a composition containing Diclofenac sodium salt 3% but not including any form of hyaluronic acid. (Number 112) The trial was conducted with 60 patients who were randomly assigned to test preparations number 109 or 112. The trial has not been completed as yet but so far 31 patients have finished the protocol. Patients were diagnosed:
4 Rheumatoid arthritis of the knee
8 Myofascial trigger points in the M.trapezius area
12 Periarthropathies of knee without effusion
7 Periarthropathies with effusion in the knee joint

The 31 patients were aged 22-75 years (27 females, 4 males). All patients were hospitalized. Patients entering the trial were thoroughly examined and type of extraarticular or articular rheumatism assessed.

On day 1 baseline pain was assessed on the 10 cm visual analogue scale (VAS) and pain measurement of the quantititative pain sensitivity using a pressure tolerance meter (PTM) were performed. Then test gel - approximately 2.g - was massaged on to the skin of maximum pain. Gels were applied 3 times daily.

0.5, 1, 1.5, and 2 hours after morning application measurements of pain sensitivity were carried out and values recorded.

This procedure was countinued on day 2, 3 and 4; measurements (VAS and PTM) of pain severity were done on day 1, 2 and 4.

Prior of the beginning of the study and at the end on day 4, physician's global assessment, assessment of swelling, tenderness and limitation of movement were recorded.

As the study is ongoing statistical evaluation is not yet available. For further details see Table 1.

**TABLE 1**

| | Composition | Composition |
|---|---|---|
| Reaction | 109, n = 16 | 112, n - 15 |
| Good Alleviation of pain | 13 | 8 |
| Moderate Alleviation of pain | 2 | 2 |
| No Alleviation of pain | 1 | 5 |

From the data recorded we have concluded that the patients to whom composition 109 was administered did better in terms of earlier and longer lasting analgesic effect (up to 4 hours) than the 112 composition especially in patients with myofascial trigger points and with periarthropathies of the knee joints without effusions. Neither composition 109 nor composition 112 treated patients showed any effect on swelling if any swelling exist at all. Systemic side effects have not been observed; one patient to whom composition 112 was administered showed reddening of the skin on the site of application.

Any intake of system NSAIDS,corticosteroids and other analgesics was not allowed one week before and during the trial.

### EXAMPLES

The following examples are offered to illustrate uses of Applicants' invention.

### Example 1

A male patient had a number of lesions (basal cell carcinoma), including one on his forehead which was a combination of major "horny epithelium" and some degree of ulceration. After continuous treatment with Formulation 1 (several times per day for several weeks of dosage amounts squeezed from tubes as ribbons of composition), the lesions showed epithelialization, no hemorrhagic areas, and no initiated areas (as they were in the past without our treatment). The "horny epithelium" and ulceration of the forehead lesion were also gone. The patient had a complete successful response with the formulation. All basal cell carcinoma lesions had been resolved and disappeared. There has been no recurrence.

### Example 2

60 year old male tennis player had sore elbow and basal cell carcinoma on forearm proximate sore elbow. Patient tried Formulation 1 to abate pain in tennis elbow. (Dr. Falk was not treating this patient for anything at the time, did not know of the basal cell proximate the elbow and merely offered the formulation for pain relief of the elbow instucting the patient to squeeze a ribbon of the composition and apply and rub into the sore elbow). However, the formulation "spilled" over onto the Patient's basal cell carcinoma. Patient was planning to have basal cell carcinoma removed surgically by another doctor, but when the patient returned to see the doctor, the basal cell carcinoma was disappearing (because of spill-over of Formulation 1). Dr. Falk was then advised and treatment was now undertaken by Dr. Falk with direct application of Formulation 1 to the lesion 3 times a day for two additional weeks. After two weeks, the basal cell carcinoma disappeared. There has been no recurrence.

### Example 3

Male, mid to late 40's had severe basal cell carcinoma on left temple. Doctors recommended its removal by surgery. However, the surgery would have been risky because of the lesion's proximity to facial nerves.

Patient saw Dr. Falk who gave him Formulation 2 to be applied in dosage amounts 3 times daily.

After 14 days, 75% of the lesion was gone. Surgery was postponed and the treatment was continued. Application of dosage amounts of Formulation 2 was continued for an additional two weeks. At the end of the 2- week period, the lesion was completely resolved and disappeared without any surgery being required. There has been no recurrence.

### Example 4

Male, early 40's, had recurrent actinic keratoses lesion on his right temple. Early attempts at removal by third parties involved the application of liquid nitrogen (twice) without final resolution. The lesion kept recurring. The patient was sent to Dr. Falk who treated the lesion with Formulation 1 with applications of dosage amounts 3 times daily for 7 days. After 7 days, the lesion was completely resolved with no subsequent recurrence.

### Example 5

A male patient with basal cell carcinoma was first treated by an oncologist who attempted to surgically excise the lesion (without success) and then irradiated the lesion again without success. The patient then attended before Dr. Falk who applied Applicant's formulation (diclofenac with sodium hyaluronate and excipients). Application was made three times daily for about a month and the lesion disappeared. Some excoriation anterior and slightly superior developed over the last two weeks but was cleared by the application of hyaluronic acid by itself.

This resolution clearly indicates that even with prior applications of unsuccessful therapies (surgery and irradiation), Applicant's formulations can be used successfully.

### Example 6

In another patient, a drug (methotrexate) was carried in hyaluronic acid and applied topically to a patient with psoriasis. The formulation was absorbed and the psoriasis cleared.

### Example 7

A patient with dermal (skin) metastases in a fibratic scar form and metastatic cancer in the form of musculoskeletal involvement in her thorax.

On topical application of our formulation comprising diclofenac (Voltaren) in hyaluronic acid (sodium hyaluronate), her pain decreased dramatically and her skin and boney involvements steadily improved.

### TOPICAL DICLOFENAC ACID 3% IN HYALURONIC ACID GEL (2.5%) BASE

A practitioner reviewed the effectiveness of topical Diclofenac Acid 3% in hyaluronic acid gel (2.5%) base in acute traumatic injuries of no longer than 3 days duration. The cases were all in the spectrum of ages between 18 and 65. Normal exclusion criteria were followed regarding exclusion of pregnancy, aspirin or N.S.A.I.D., allergies or active peptic ulceration.

As an overall, the following impressions were gained from 30 cases:
1. The topical H.D.(composition comprising sodium hyaluronate and diclofenac) had an obvious analgesic action with onset occurring rapidly within one hour; this is a phenomenon not obviously seen with other non-steroidals that we have used.
2. There was a very definite patient acceptance of the gel as a form of treatment, being logical, easy to apply, without local or systemic side effects, rapid absorption with no staining of clothing.
3. The anti-inflammatory action was equivalent on a "guestimate" based on experience of similar injuries to oral N.S.A.I.D.s, without the threat or risk of side effects.

In summary, compared with other topical N.S.A.I.D.s the analgesic effect is distinct, the anti-inflammatory is equal to oral N.S.A.I.D.s and the patients' acceptance is far superior to any other diclofenac or piroxicam topical that the practitioner evaluated.

Following the practitioner's basic preamble regarding the parallelism of topical N.S.A.I.D.s and topical steroids,the practitioner has used the former in contact dermatitis, insect bites and U.V. erothema, all with very positive effects, again pointing direction to trials of a double blind nature in these fields.

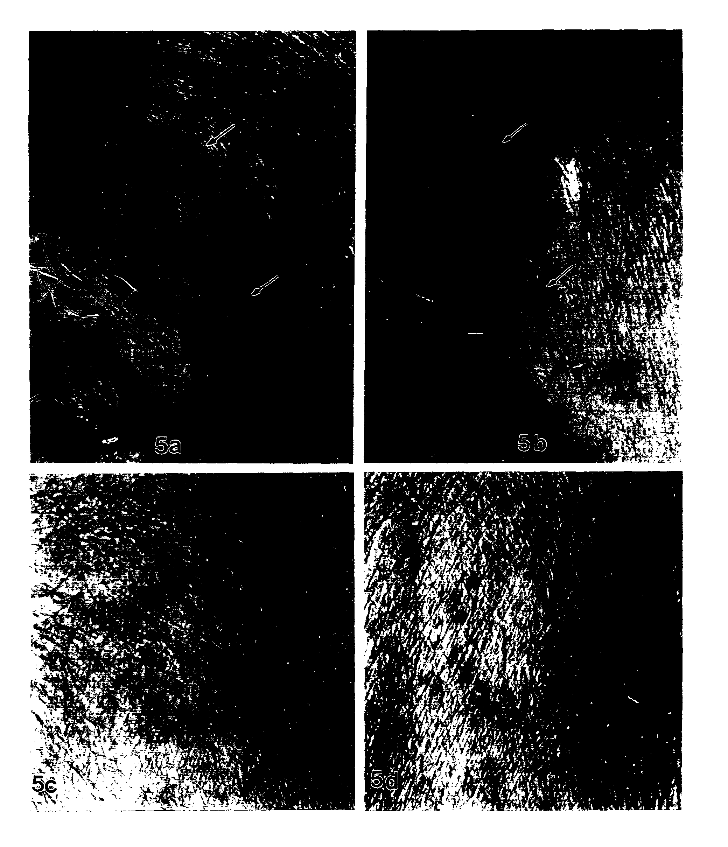

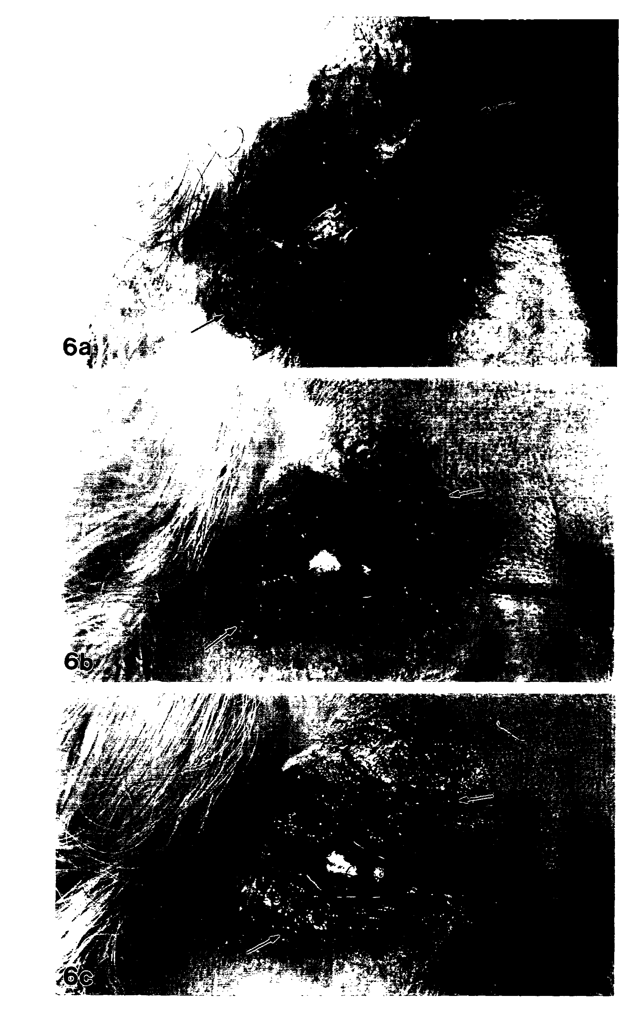

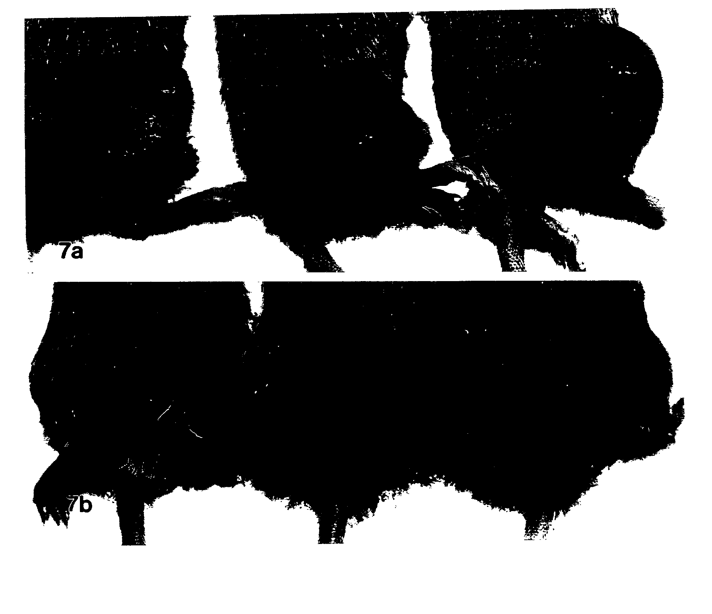

Photographs were taken of patients with basal cell carcinoma Figures 1-6 photographs, and of mice with tumors induced in the skin of the hind legs (Figure 7 photographs). The patients were treated by using combinations of NSAIDS, (non-steroidal anti-inflammatory drugs) and hyaluronic acid (including sodium hyaluronate) according to the invention (3% diclofenac in 2.5% sodium hyaluronate gel base). Each of the six sets of Figures made up of photographs of the different persons should include a legend describing or explaining each picture as follows:
Legend for Figures 1A and 1B should read:
   - Patient :: W.D., male, 82 years
   - Diagnosis:: Basal cell carcinoma
   - Treatment:: NSAIDS plus HA gel. 3 times per day
   - Figure 1A:: June, 1991
   - Figure 1B:: December, 1991
Legend for Figures 2A and 2B should read:
   - Patient :: M.F., male, 45 years
   - Diagnosis:: Basal cell carcinoma
   - Treatment:: NSAIDS plus HA gel. 3 times per day
   - Figure 2A:: January, 1992
   - Figure 2B:: April, 1992
Legend for Figures 3A, 3B, 3C and 3D should read:
   - Patient :: H.A., male, 82 years
   - Diagnosis:: Basal cell carcinoma
   - Treatment:: NSAIDS plus HA gel. 3 times per day
   - Figure 3A:: January 26, 1992
   - Figure 3B:: March 16, 1992
   - Figure 3C:: January 26, 1992
   - Figure 3D:: March 16,1992
Legend for Figures 4A, 4B, 4C and 4D should read:
   - Patient :: R.F., male, 64 years
   - Diagnosis:: Basal cell carcinoma
   - Treatment:: NSAIDS plus HA gel. 3 times per day
   - Figure 4A:: January 26, 1992
   - Figure 4B:: March 16, 1992
   - Figure 4C:: January 26, 1992
   - Figure 4D:: March 16, 1992
Legend for Figures 5A, 5B, 5C and 5D should read:
   - Patient :: R.W., male, 86 years
   - Diagnosis:: Basal cell carcinoma
   - Treatment:: NSAIDS plus HA gel. 3 times per day
   - Figure 5A:: January 26, 1992
   - Figure 5B:: March 16, 1992
   - Figure 5C:: January 26, 1992 untreated
   - Figure 5D:: March 16, 1992 untreated
Legend for Figures 6A, 6B and 6C should read:
   - Patient :: E.D., female, 70 years
   - Diagnosis:: Basal cell carcinoma
   - Treatment:: NSAIDS plus HA gel. 3 times per day
   - Figure 6A:: April 20, 1992
   - Figure 6B:: May 13, 1992
   - Figure 6C:: July 7, 1992
The Legend for Figure 7 (Figures 7A and 7B) relate to:
   - Mouse Strain:: DBA₂
   - Tumour:: p815
   - Figure 7A:: control, 19 days
   - Figure 7B:: Novantrone plus HA gel 19 days

The mice shown in Figures 7A and 7B had tumours induced in the skin of their hind legs and dosage amounts (2ml) of Novatrone (10 mg. per dosage amount) (MITOXANTRONE (t.m.) and 2.5% sodium hyaluronate were applied (rubbed onto) the skin at the site of the pathology. The tumours reduced in size (See Figure 7B) clearly illustrating the percutaneous delivery of the medicine by the hyaluronic acid. (See Figure 7).

The following additional comments are made with respect to the patients.

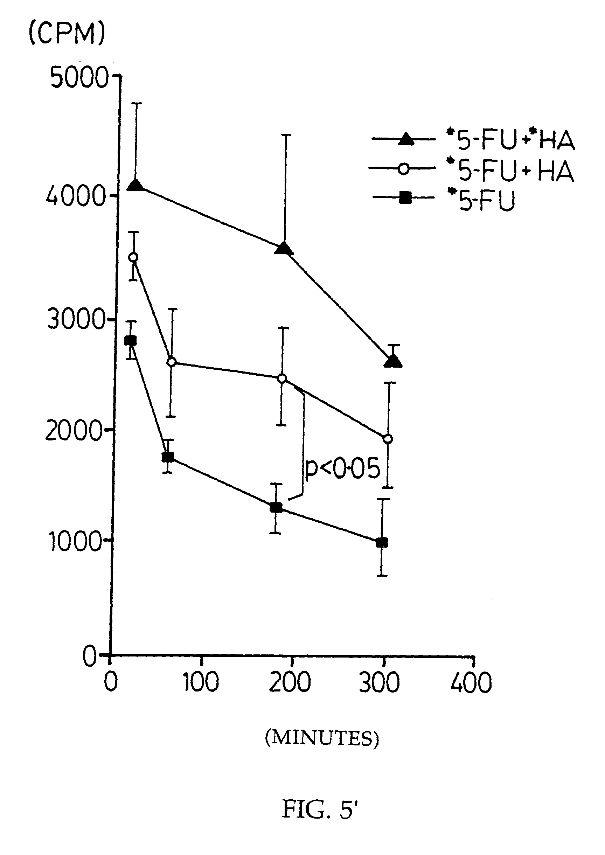

With respect to R.W. and Figure 5, the reader will note in Figures 5a and 5c the patient suffered from basal cell carcinoma on his back (Figure 5c) and his temple (Figure 5a). Because of the age of the individual (86) the basal cell carcinoma on his back could not be reached by him for application of the medication. Thus the basal cell carcinoma in 5c remained untreated and grew (see Figure 5d). However, the portion indicated in 5a on his temple could be reached and after application of the basal cell carcinoma formulation to the temple and forehead the results are as in 5b; the basal cell carcinoma is disappearing. Thus, the gentlemen's own method of treatment acted as a control.

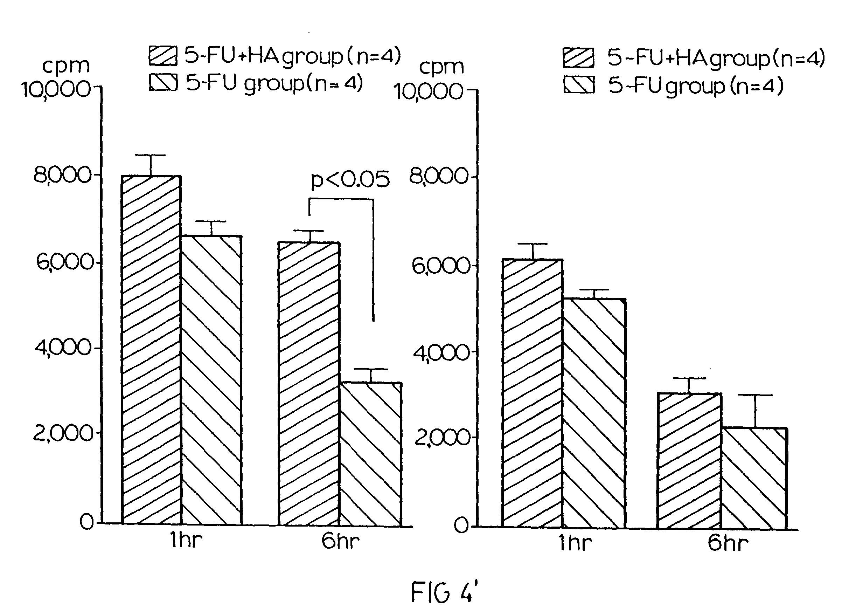

With respect to R.F. and Figures 4, two areas of basal cell carcinoma in need of treatment are shown by the arrows in Figures 4a and 4c and the results are shown in Figures 4b and 4d as indicated by the arrows after treatment with Applicant's invention.

With respect to H.A., male, and Figures 3, Figures 3 indicates two areas of basal cell carcinoma by the arrows, close-ups of which are shown in Figures 3a and 3c. After treatment with the NSAIDS and HA gel three times a day for the period between January 26, 1992 and March 16, 1992 the basal cell carcinoma is clearing as per Figures 3b and 3d.

The same is true with respect to male M.F. and Figures 2 which appears clear in the photographs (see Figure 2a and the response shown in Figure 2b).

With respect to male, W.D. and Figures 1, the upper lesion in Figure 1a (indicated by the upper arrow) is gone after treatment with Applicant's invention (See Figure 1b) and the two lower lesions in Figure 1a are well on their way to disappearing (See Figure 1b).

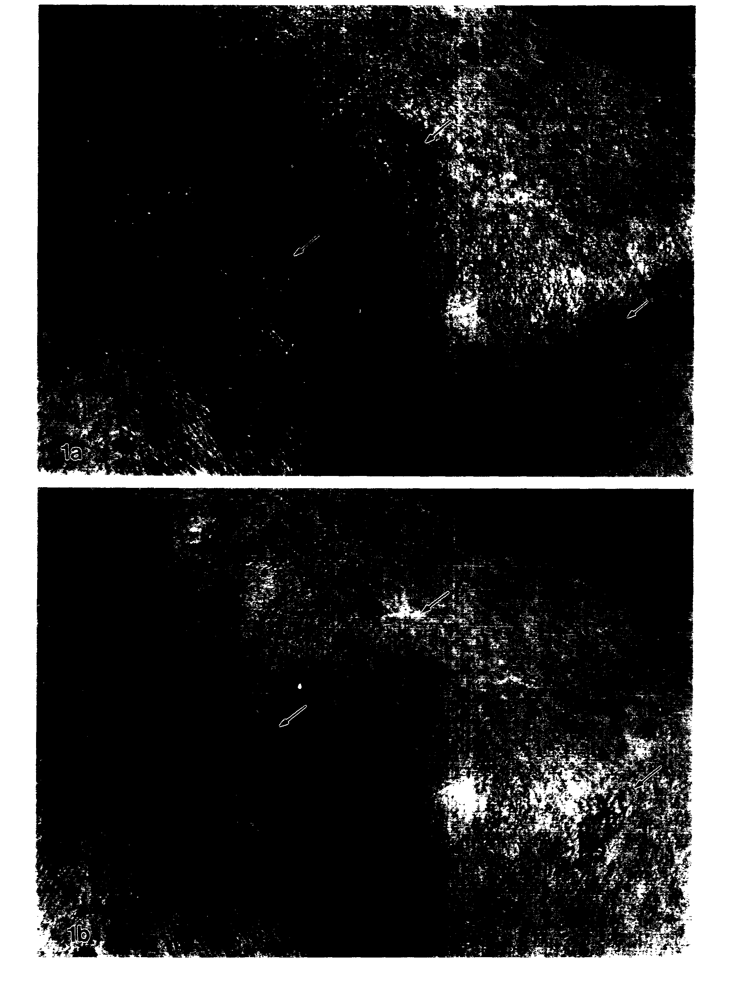

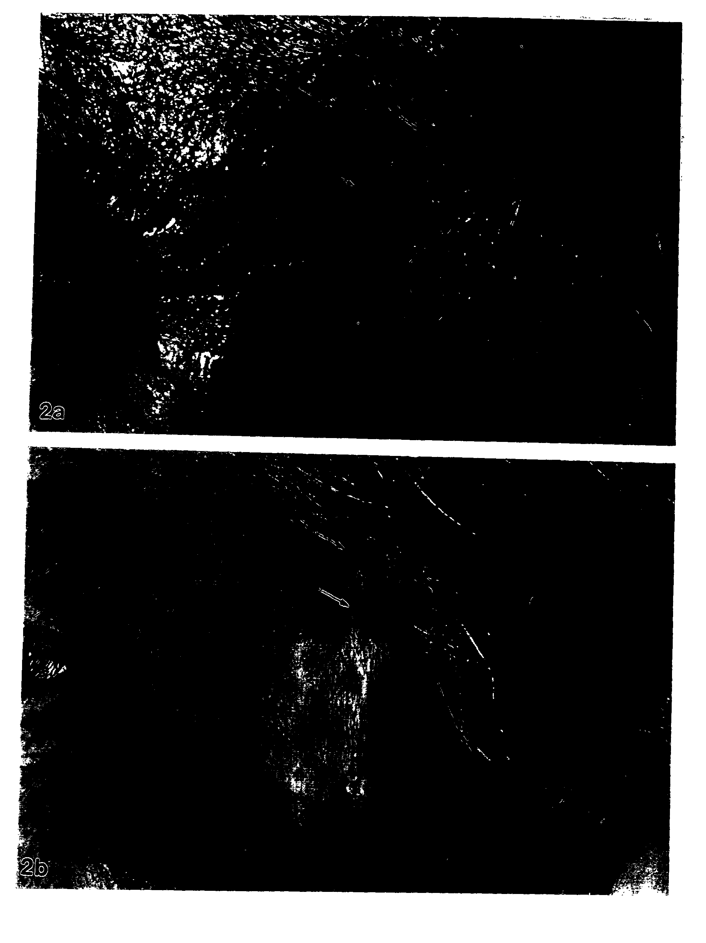

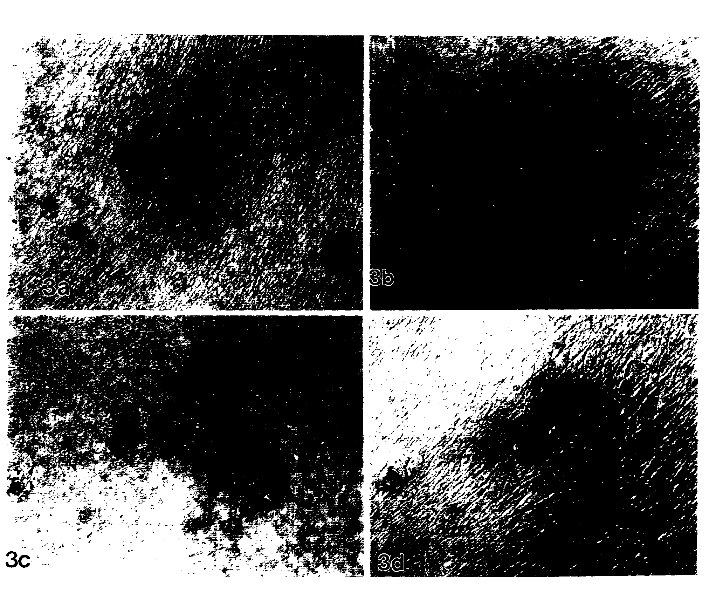

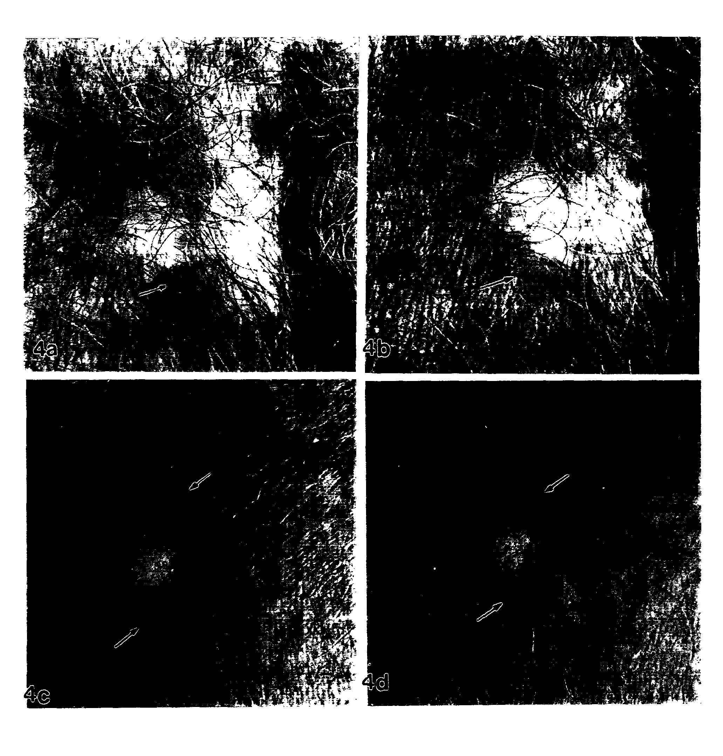

With respect to female D and Figure 6, the lesion was left untreated for a long period and gradually encompassed her eye. Surgery could not be undertaken without jeopardizing the eye. By applying Applicant's invention (dosage amounts) over a prolonged period, the basal cell carcinoma has constantly decreased in size.

With respect to Figure 7, (7a) shows mice having tumors in the skin induced in their hind legs. After continuous applications to the shaved hind legs having the tumors in the skin by rubbing in dosage amounts by Applicant's invention, the tumors have decreased in size. (See Figure 7b)

| Radioactivity in Tumour Tissue | | | |
|---|---|---|---|
| | | | Tumour Tissue |
| 15 min | 3H-5FU | (n=6) | 2810±165 |
| | 3H-5FU + HA | (n=6) | 352±190 |
| | 3H-5FU + 3H-HA | (n=4) | 4087±681 |
| 60 min | 3H-5FU | (n=3) | 1751±149 |
| | 3H-5FU + HA | (n=4) | 2599±489 |
| 3 hrs | 3H-5FU | (n=6) | 1493±227 |
| | 3H-5FU + HA | (n=6) | 2512±449 |
| | 3H-5FU + 3H-HA | (n=4) | 3606±929 |
| 5 hrs | 3H-5FU | (n=3) | 853±129 |
| | 3H-5FU + HA | (n=3) | 1981±479 |
| | 3H-5FU + 3H-HA | (n=3) | 2168±163 |
| mean±S.E. HA: 15 mg/kg (30 µCi/kg) 5-FU: 20 mg/kg (30 µCi/kg) | | | |

See Figure 5' of page 5/12 of the Figures which comprises a graph entitled "RADIOACTIVITY IN TUMOUR TISSUE" comparing CPM on the vertical with time in Minutes on the horizontal (for example 100, 200, 300).
1. Radioactivity in tumour tissue in 5-FU + HA group is higher than that in 5-FU group. There is significant difference (p>0.05, ANOVA) between with and without HA at 3 hrs after injection. The high intratumour concentration was retained for a prolonged time in 5-FU+HA group. (This retention was confirmed by the intratumour injection study.)
2. These results teach that HA can enhance 5-FU uptake in tumour tissue. This phenomenon results from HA distribution (in tumour tissue HA may be lost from the extracellular matrix) and the vascular uniqueness of tumour tissue (hyperpermiability of tumour vessels to macromolecular drug, HA).

## Claims

1. The use of:
(1) a medicinal and/or therapeutic agent in an amount effective to therapeutically treat a disease or condition of the skin and/or exposed tissue of a human, and
(2) hyaluronic acid and/or salts thereof and/or homologues, analogues, derivatives, complexes, esters, fragments, and subunits of hyaluronic acid, for the manufacture of a pharmaceutical composition for the topical treatment of a disease or a condition of the skin and/or exposed tissue,
**characterized in that** said composition is to be topically applied in a dosage amount in which component (2) exceeds 5 mg/cm² of the skin and/or exposed tissue to which the dosage amount is to be applied and is in such form that component (2) is immediately available to transport component (1) percutaneously into the epidermis of the skin or exposed tissue to the site of trauma and/or pathology of the disease or condition to be treated in the skin or exposed tissue.

2. The use of claim 1 wherein the hyaluronic acid and/or salts thereof and/or homologues, analogues, derivatives, complexes, esters, fragments, and/or subunits of hyaluronic acid is hyaluronic acid and/or a salt thereof.

3. The use of claim 2 wherein the hyaluronic acid and/or salt thereof is sodium hyaluronate.

4. The use of claim 1, 2 or 3 wherein the molecular weight of the form of hyaluronic acid is less than 750,000 daltons.

5. The use of claim 1, 2, 3 or 4 wherein the disease and/or condition of the skin and/or exposed tissue at the site of the trauma and/or pathology is selected from at least one of basal cell carcinoma, the precancerous, often recurrent, actinic keratoses lesions, fungal lesions, "liver" spots, squamous cell tumours, metastatic cancer of the breast to the skin, primary and metastatic melanoma in the skin, malignancies and/or tumours in the skin, genital warts (condyloma acuminata), cervical cancer, and HPV (Human Papilloma Virus) including HPV of the cervix, psoriasis (both plaque-type psoriasis and nail bed psoriasis), corns on the feet and hair loss on the head of pregnant women.

6. The use of claim 1, 2, 3, 4 or 5 wherein the medicine and or therapeutic agent comprises an effective non-toxic dosage amount which inhibits prostaglandin synthesis.

7. The use of claim 6 wherein the medicine and/or therapeutic agent is a non-steroidal anti-inflammatory drug (NSAID).

8. The use of claim 1, 2, 3, 4, or 5 wherein the medicine and/or therapeutic agent is an anti-cancer drug,

9. The use of claim 8 wherein the anti-cancer drug is selected from novantrone.

10. The use of claim 7 wherein the NSAID is selected from diclofenac, diclofenac sodium indomethacin, naproxen, and (+/-) tromethamine salt of ketorolac.

11. The use of claim 7 wherein the NSAID is selected from Ibuprofen, Piroxicam, Propionic Acid derivatives, acetylsalicylic acid and Flunixin.

12. The use of any preceding claim wherein component (2) is sodium hyaluronate having a molecular weight of between 150,000 daltons and 225,000 daltons.

13. The use of claim 7 or 12 wherein the NSAID is diclofenac sodium.

14. The use of claim 3, 4 or 5 wherein component (1) is diclofenac sodium and the concentration of the diclofenac sodium is 3% by weight of the pharmaceutical composition, component (2) is sodium hyaluronate in a concentration of 2½% by weight of the pharmaceutical composition having a molecular weight of 661,600 daltons and the pharmaceutical composition further comprises glycerine 5% by weight of the composition, benzyl alcohol 3% by weight of the composition and sterile water.

15. The use of any preceding claim wherein the pharmaceutical composition comprises suitable topical excipients.

16. The use of any preceding claim wherein component (2) in each dosage amount is in an amount of at least 10 mg/cm² of skin and/or exposed tissue to which it is to be applied.

17. The use of claim 1, 2, 3 or 5 for the treatment of pain wherein component (2) of each dosage amount is in an amount of at least 20 mg/cm² of the skin and/or exposed tissue to which it is to be applied and component (1) is an NSAID.

18. The use of claim 1, 2, 3 or 4 for the treatment of pain wherein component (2) of each dosage amount is an amount of at least 10 mg/cm² of skin and/or exposed tissue to which it is to be applied and component (1) is an NSAID.

19. The use of claim 5 wherein the disease and/or condition is basal cell carcinoma.

20. The use of claim 5 wherein the disease or condition is actinic keratoses.

21. The use of claim 5 wherein the disease or condition is liver spots.

22. The use of claim 5 wherein the disease or condition is squamous cell tumours.

23. The use of claim 5 wherein the disease or condition is metastatic cancer of the breast to the skin.

24. The use of claim 5 wherein the disease or condition is primary and metastatic melanoma of the skin.

25. The use of claim 5 wherein the disease or condition is malignancies and/or tumour of the skin.

26. The use of claim 5 wherein the disease or condition is genital warts (condyloma acuminata).

27. The use of claim 5 wherein the disease or condition is cervical cancer.

28. The use of claim 5 wherein the disease or condition is Human Papilloma Virus (HPV).

29. The use of claim 5 wherein the disease or condition is psoriasis.

30. The use of claim 5 wherein the disease or condition is corns on the feet or the hair loss on the head of pregnant women.

## Patentansprüche

1. Verwendung:
(1) eines medizinischen und/oder therapeutischen Wirkstoffes in einer für die therapeutische Behandlung einer Krankheit oder eines Leidens der Haut und/oder exponierten Gewebes eines Menschen wirksamen Menge, und
(2) von Hyluronsäure und/oder Salzen davon und/oder Homologen, Analoga, Derivaten, Komplexen, Estern, Fragmenten und Untereinheiten von Hyluronsäure zur Herstellung einer pharmazeutischen Zusammensetzung für die topische Behandlung einer Krankheit oder eines Leidens der Haut und/oder von exponiertem Gewebe,
**dadurch gekennzeichnet, dass** die Zusammensetzung topisch in einer Dosiermenge anzuwenden ist, in welcher der Bestandteil (2) 5 mg/cm² der Haut und/oder des exponierten Gewebes übersteigt, auf der bzw. dem die Dosiermenge anzuwenden ist, und dadurch, dass die Zusammensetzung so gestaltet ist, dass der Bestandteil (2) unmittelbar zur Verfügung steht, um den Bestandteil (1) perkutan in die Epidermis der Haut oder des exponierten Gewebes zur Stelle des Traumas und/oder der Pathologie der Krankheit oder des Leidens zu transportieren, die in der Haut oder dem exponierten Gewebe zu behandeln sind.

2. Verwendung nach Anspruch 1, wobei die Hyluronsäure und/oder Salze davon und/oder Homologe, Analoga, Derivate, Komplexe, Ester, Fragmente und/oder Untereinheiten der Hyluronsäure Hyluronsäure und/oder ein Salz davon sind.

3. Verwendung nach Anspruch 2, wobei die Hyluronsäure und/oder ein Salz davon Natriumhyaluronat ist.

4. Verwendung nach Anspruch 1, 2 oder 3, wobei das Molekulargewicht der Form der Hyluronsäure weniger als 750.000 Dalton beträgt.

5. Verwendung nach Anspruch 1, 2, 3 oder 4, wobei die Krankheit und/oder das Leiden der Haut und/oder des exponierten Gewebes an der Stelle des Traumas und/oder der Pathologie ausgewählt wird aus mindestens einem von Basalzellkarzinom, den prämalignen, oft wiederkehrenden, Läsionen aktinischer Keratose, Pilzläsionen, "Leber"-Flecken, Plattenepithelkarzinomen, zur Haut metastasierendem Brustkarzinom, primären und metastasierenden Hautmelanomen, bösartigen Geschwulsten und/oder Tumoren in der Haut, Warzen an Geschlechtsteilen (Condyloma acuminata), Gebärmutterhalskrebs und HPV (Humanes Papillomavirus), einschließlich HPV der Zervix, Psoriasis (sowohl Plaque-Psoriasis als auch Nagelbettpsoriasis), Hühneraugen an den Füßen und Haarausfall auf dem Kopf von schwangeren Frauen.

6. Verwendung nach Anspruch 1, 2, 3, 4 oder 5, wobei das Medikament und/oder der therapeutische Wirkstoff eine wirksame nicht-toxische Dosierungsmenge umfasst, die Prostaglandin-Synthese hemmt.

7. Verwendung nach Anspruch 6, wobei das Medikament und/oder der therapeutische Wirkstoff ein nicht-steroidales entzündungshemmendes Arzneimittel (NSAID) ist.

8. Verwendung nach Anspruch 1, 2, 3, 4 oder 5, wobei das Medikament und/oder der therapeutische Wirkstoff eine krebsbekämpfende Substanz ist.

9. Verwendung nach Anspruch 8, wobei die antineoplastische Substanz aus Novantron ausgewählt wird.

10. Verwendung nach Anspruch 7, wobei das NSAID aus Diclofenac, Diclofenac-Natrium-Indomethacin, Naproxen und (+/-) Tromethaminsalz von Ketorolac ausgewählt wird.

11. Verwendung nach Anspruch 7, wobei das NSAID aus Ibuprofen, Piroxicam, Propionsäurederivaten, Acetylsalicylsäure und Flunixin ausgewählt wird.

12. Verwendung nach einem der vorhergehenden Ansprüche, wobei der Bestandteil (2) Natriumhyaluronat mit einem Molekulargewicht zwischen 150.000 Dalton und 225.000 Dalton ist.

13. Verwendung nach Anspruch 7 oder 12, wobei das NSAID Diclofenac-Natrium ist.

14. Verwendung nach Anspruch 3, 4 oder 5, wobei Bestandteil (1) Diclofenac-Natrium ist und die Konzentration des Diclofenac-Natriums bei 3 Gew% der pharmazeutischen Zusammensetzung liegt, wobei Bestandteil (2) Natrium-Hyaluronat in einer Konzentration von 2½ Gew.-% der pharmazeutischen Zusammensetzung mit einem Molekulargewicht von 661.600 Dalton ist und die pharmazeutische Zusammensetzung ferner Glyzerin im Ausmaß von 5 Gew% der Zusammensetzung, Benzylalkohol im Ausmaß von 3 Gew% der Zusammensetzung und steriles Wasser umfasst.

15. Verwendung nach einem der vorhergehenden Ansprüche, wobei die pharmazeutische Zusammensetzung geeignete topische Arzneimittelträger umfasst.

16. Verwendung nach einem der vorhergehenden Ansprüche, wobei Bestandteil (2) bei jeder Dosiermenge in einem Mengenbereich von mindestens 10 mg/cm² der Haut und/oder des exponierten Gewebes liegt, auf die bzw. das er anzuwenden ist.

17. Verwendung nach Anspruch 1, 2, 3 oder 5 zur Schmerzbehandlung, wobei Bestandteil (2) bei jeder Dosiermenge in einem Mengenbereich von mindestens 20 mg/cm² der Haut und/oder des exponierten Gewebes liegt, auf die bzw. das er anzuwenden ist, und wobei Bestandteil (1) ein NSAID ist.

18. Verwendung nach Anspruch 1, 2, 3 oder 4 zur Schmerzbehandlung, wobei Bestandteil (2) bei jeder Dosiermenge in einem Mengenbereich von mindestens 10 mg/cm² der Haut und/oder des exponierten Gewebes liegt, auf die bzw. das er anzuwenden ist, und wobei Bestandteil (1) ein NSAID ist.

19. Verwendung nach Anspruch 5, wobei die Krankheit und/oder das Leiden ein Basalzellkarzinom ist.

20. Verwendung nach Anspruch 5, wobei die Krankheit oder das Leiden aktinische Keratose ist.

21. Verwendung nach Anspruch 5, wobei die Krankheit oder das Leiden Leberflecken sind.

22. Verwendung nach Anspruch 5, wobei die Krankheit oder das Leiden Plattenepithelkarzinome sind.

23. Verwendung nach Anspruch 5, wobei die Krankheit oder das Leiden ein zur Haut metastasierendes Brustkarzinom ist.

24. Verwendung nach Anspruch 5, wobei die Krankheit oder das Leiden ein primäres und metastasierendes Hautmelanom ist.

25. Verwendung nach Anspruch 5, wobei die Krankheit oder das Leiden bösartige Geschwulste und/oder Hauttumore sind.

26. Verwendung nach Anspruch 5, wobei die Krankheit oder das Leiden Warzen an Geschlechtsteilen (Condyloma acuminata) sind.

27. Verwendung nach Anspruch 5, wobei die Krankheit oder das Leiden Gebärmutterhalskrebs ist.

28. Verwendung nach Anspruch 5, wobei die Krankheit oder das Leiden das Humane Papillomavirus (HPV) ist.

29. Verwendung nach Anspruch 5, wobei die Krankheit oder das Leiden Psoriasis ist.

30. Verwendung nach Anspruch 5, wobei die Krankheit oder das Leiden Hühneraugen an den Füßen oder Haarausfall auf dem Kopf von schwangeren Frauen sind.

## Revendications

1. Utilisation
(1) d'un agent médicinal et/ou thérapeutique en une quantité efficace pour un traitement thérapeutique d'une maladie ou d'un état de la peau et/ou d'un tissu exposé chez l'homme et
(2) d'acide hyaluronique et/ou de sels de celui-ci et/ou d'homologues, analogues, dérivés, complexes, esters, fragments et sous-unités d'acide hyaluronique, pour la fabrication d'une composition pharmaceutique pour le traitement topique d'une maladie ou d'un état de la peau et/ou d'un tissu exposé,
**caractérisée en ce que** ladite composition est destinée à une application topique en une quantité posologique dans laquelle le composant (2) dépasse 5 mg/cm² de peau et/ou de tissu exposé sur lequel la quantité posologique est destinée à une application et est sous une forme telle que le composant (2) est immédiatement disponible pour transporter le composant (1) par voie percutanée dans l'épiderme de la peau ou du tissu exposé jusqu'au site de lésion et/ou de pathologie de la maladie ou de l'état à traiter de la peau ou du tissu exposé.

2. Utilisation suivant la revendication 1, dans laquelle l'acide hyaluronique et/ou des sels de celui-ci et/ou des homologues, analogues, dérivés, complexes, esters, fragments et/ou sous-unités d'acide hyaluronique est de l'acide hyaluronique et/ou un sel de celui-ci.

3. Utilisation suivant la revendication 2, dans laquelle l'acide hyaluronique et/ou un sel de celui-ci est du hyaluronate de sodium.

4. Utilisation suivant la revendication 1, 2 ou 3, dans laquelle le poids moléculaire de la forme de l'acide hyaluronique est inférieur à 750 000 daltons.

5. Utilisation suivant la revendication 1, 2, 3 ou 4, dans laquelle la maladie et/ou l'état de la peau et/ou du tissu exposé au site de la lésion et/ou de la pathologie est sélectionné(e) parmi au moins un parmi un carcinome basocellulaire, des lésions de kératose actiniques précancéreuses souvent récurrentes, des lésions fongiques, du lentigo sénile, des tumeurs de cellules d'épithélium pavimenteux, un cancer de métastases du sein vers la peau, un mélanome primaire et de métastases de la peau, des affections malignes et/ou des tumeurs de la peau, des verrues génitales (condylome acuminé), un cancer du col, un HPV (virus du papillome humain) notamment un HPV du col, le psoriasis (à la fois le psoriasis de type en plaques et le psoriasis du lit de l'ongle), des cors aux pieds et une perte de cheveux chez les femmes enceintes.

6. Utilisation suivant la revendication 1, 2, 3, 4 ou 5, dans laquelle le médicament et/ou l'agent thérapeutique comprend une quantité posologique efficace non toxique qui inhibe une synthèse de prostaglandines.

7. Utilisation suivant la revendication 6, dans laquelle le médicament et/ou l'agent thérapeutique est un médicament anti-inflammatoire non stéroïdien (NSAID).

8. Utilisation suivant la revendication 1, 2, 3, 4 ou 5, dans laquelle le médicament et/ou l'agent thérapeutique est un médicament anticancéreux.

9. Utilisation suivant la revendication 8, dans laquelle le médicament anticancéreux est sélectionné parmi la novantrone.

10. Utilisation suivant la revendication 7, dans laquelle le NSAID est sélectionné parmi du diclofénac, du diclofénac forme sodique, de l'indométhacine, du naproxène et le sel de (+/-)-trométhamine du kétorolac.

11. Utilisation suivant la revendication 7, dans laquelle le NSAID est sélectionné parmi l'ibuprofen, le piroxicam, des dérivés de l'acide propionique, l'acide acétylsalicylique et le flunixin.

12. Utilisation suivant l'une quelconque des revendications précédentes, dans laquelle le composant (2) est du hyaluronate de sodium présentant un poids moléculaire entre 150 000 daltons et 225 000 daltons.

13. Utilisation suivant la revendication 7 ou 12, dans laquelle le NSAID est du diclofénac forme sodique.

14. Utilisation suivant la revendication 3, 4 ou 5, dans laquelle le composant (1) est du diclofénac forme sodique et la concentration du diclofénac forme sodique est de 3% en poids de la composition pharmaceutique, le composant (2) est du hyaluronate de sodium en une concentration de 2,5% en poids de la composition pharmaceutique présentant un poids moléculaire de 661 600 daltons et la composition pharmaceutique comprend en outre du glycérol à 5% en poids de la composition, de l'alcool benzylique à 3% en poids de la composition et de l'eau stérile.

15. Utilisation suivant l'une quelconque des revendications précédentes, dans laquelle la composition pharmaceutique comprend des excipients topiques appropriés.

16. Utilisation suivant l'une quelconque des revendications précédentes, dans laquelle le composant (2) dans chaque quantité posologique est en une quantité d'au moins 10 mg/cm² de peau et/ou de tissu exposé sur lequel il est destiné à être appliqué.

17. Utilisation suivant la revendication 1, 2, 3 ou 5, pour le traitement de la douleur, dans laquelle le composant (2) de chaque quantité posologique est en une quantité d'au moins 20 mg/cm² de peau et/ou de tissu exposé sur lequel il est destiné à être appliqué et le composant (1) est un NSAID.

18. Utilisation suivant la revendication 1, 2, 3 ou 4, pour le traitement de la douleur dans laquelle le composant (2) de chaque quantité posologique est une quantité d'au moins 10 mg/cm² de peau et/ou de tissu exposé sur lequel il est destiné à être appliqué et le composant (1) est un NSAID.

19. Utilisation suivant la revendication 5, dans laquelle la maladie et/ou l'état est un carcinome basocellulaire.

20. Utilisation suivant la revendication 5, dans laquelle la maladie ou l'état comprend des kératoses actiniques.

21. Utilisation suivant la revendication 5, dans laquelle la maladie ou l'état est du lentigo sénile.

22. Utilisation suivant la revendication 5, dans laquelle la maladie ou l'état comprend des tumeurs de cellules d'épithélium pavimenteux.

23. Utilisation suivant la revendication 5, dans laquelle la maladie ou l'état est un cancer de métastases du sein vers la peau.

24. Utilisation suivant la revendication 5, dans laquelle la maladie ou l'état est un mélanome primaire et de métastases de la peau.

25. Utilisation suivant la revendication 5, dans laquelle la maladie ou l'état comprend des affections malignes et/ou une tumeur de la peau.

26. Utilisation suivant la revendication 5, dans laquelle la maladie ou l'état comprend des verrues génitales (condylome acuminé).

27. Utilisation suivant la revendication 5, dans laquelle la maladie ou l'état est un cancer du col.

28. Utilisation suivant la revendication 5, dans laquelle la maladie ou l'état est un virus du papillome humain (HPV).

29. Utilisation suivant la revendication 5, dans laquelle la maladie ou l'état est le psoriasis.

30. Utilisation suivant la revendication 5, dans laquelle la maladie ou l'état comprend des cors aux pieds ou une perte de cheveux chez les femmes enceintes.
